# EUROPEAN PATENT APPLICATION

(11) **EP 1 950 311 A1**
(43) Date of publication of application: **30.07.2008**
(21) Application number: 07110083.8
(22) Date of filing: 12.06.2007
(51) Int. Cl.: C12Q 1/68

(54) **Transgenic plant event detection**

(30) Priority: 29.01.2007 EP 07447008; 16.05.2007 EP 07108343
(71) Applicant: Scientific Institute of Public Health (IPH), 1050 Bruxelles (BE)
(72) Inventor: Van den Bulcke, Marc Henri Germain, B-9000 Gent (BE); Lievens, Antoon Piet Nelly Raoul, B-9000 Gent (BE); Leunda, Amaya, B-1000 Brussels (BE); Mbongolo Mbella, Etondoh Guillaume, B-1080 Brussels (BE); Sneyers, Myriam Jacqueline Sylviane, B-5030 Gembloux (BE)
(74) Representative: De Clercq, Ann G. Y.

(57) **Abstract**

The present invention relates to detection of materials derived from transgenic plant events. In particular, the invention provides methods, reagents, kits and reference materials for detecting the presence or absence in a sample of genetic material derived from and attributable to select transgenic plant events.

## Description

### FIELD OF THE INVENTION

The present invention relates to detection of materials derived from transgenic plant events. In particular, the invention provides methods, reagents, kits and reference materials for detecting the presence or absence in a sample of genetic material derived from and attributable to select transgenic plant events.

### BACKGROUND OF THE INVENTION

Genetically modified organisms (GMOs), in particular genetically modified plants, are gaining importance in agriculture as well as in the production and marketing of foods and feed.

However, due to existing concerns regarding the impact of GMOs on the environment and on consumers' health, the introduction of new GMOs commonly requires regulatory approval and, in many countries including the European Union, food and feed products containing or produced from GMOs are themselves subjected to authorisation and compulsory labelling (see, e.g., Regulation (EC) no. 1829/2003. Off J Eur Communities: Legis. 2003, L268: 1-23; Regulation (EC) no. 1830/2003. Off J Eur Communities: Legis. 2003, L268: 24-28).

Consequently, tracing of GMOs in the environment and through the cultivation process and the food or feed production chain is fundamental for environmental risk assessment, as well as necessary to preserve consumers' confidence and to satisfy or verify the observance of mandatory regulations, including GMO product labelling.

This requires the availability of methods that can determine the presence, identity and/or quantity of GMOs or materials originating or derived therefrom in, for example, food and feed sources, ingredients and/or processed consumables. DNA-based are very useful in this respect, not least because DNA often withstands physical and chemical food processing treatments better than other molecules, such as proteins. For example, real-time polymerase chain reaction (PCR) proved to be a specific and sensitive technique for the quantification of nucleic acids indicative of the presence of DNA originating from GMOs.

Assays are available to unambiguously conclude the presence or absence of genetic material derived from a select plant transformation event in a sample. Commonly, such assays can detect the presence of a unique, event-specific nucleotide sequence found in the plant transformation event of interest but absent from other events. For example, such distinctive nucleotide sequences may be present at junctions between the GMO's endogenous genomic sequence and the sequence of the transforming gene construct at the genomic insertion site of the latter; detection may for instance involve PCR amplification across the said junction using specific primers flanking the junction (see, e.g., Hernandez et al. 2003. Transgenic Res 12: 179-189; Hernandez et al. 2004. J Cereal Sci 39: 99-107; Berdal et al. 2001. Eur Food Res Technol 213: 432-438; Nielsen et al. 2004. Eur Food Res Technol 219: 421-427; or Ronning et al. 2003. Eur Food Res Technol 216: 347-354).

However, these event-specific assays can often be procured only as highly priced kits allowing for a limited number of tests. Yet, the number of generated, authorised and marketed and GMOs keeps increasing. Hence, an unambiguous determination of the GMO composition of a sample would in principle require to apply an ever-growing battery of event-specific detection assays on each sample, which is both laborious and cost demanding.

Therefore, while it is desirable to employ event-specific assays due to the unambiguous nature of their output, there exists a need for using these assays in a more effective and targeted manner, such as to improve the detection of GMOs detection, e.g., in terms of time expense, cost and labour intensity.

### SUMMARY OF THE INVENTION

In aspects, the present invention provides methods, reagents, kits and reference materials that address one or more of the above discussed needs of the art.

In general, the invention hence provides improved methods, reagents, kits and reference materials for detecting material derived from GMOs, and preferably from genetically modified plants, in samples.

More particularly, the inventors realised that by carefully choosing certain sequence features to be assayed in the sample - wherein the said sequence features need not be event-specific and may in fact be shared between two or more events - one can conclude whether the sample potentially contains material derived from one or more transformation events or, conversely, whether the sample does not contain material derived from one or more such transformation events. The assays of the invention can thus provide a valuable indication of the potential GMOs contents of the sample and thereby advantageously reduce the number of subsequent tests needed to verify the presence of material derived from one or more particular transformation events in the sample; this can in turn improve the cost-, time- and/or labour-effectiveness of GMOs detection methods. For example, the assays of the invention may allow to considerably reduce, for example, the number of event-specific assays needed to characterise the GMOs composition of the sample.

The inventors also assessed the known, commercially significant and/or authorised transformation events to select sequence features for being assayed in a sample, such as to reduce the number of individual test reactions needed per sample yet ensuring adequate informativeness of the GMO profiling according to the invention.

The present invention integrates the above relevant realisation in its diverse aspects.

Hence, in a particularly preferred aspect (herein aspect "A1") the invention concerns a method to examine a sample for the presence or absence of material derived from one or more transgenic plant events comprising the steps of:
(1) detecting the presence or absence in the sample of nucleic acids comprising or consisting of:
   a) "Zm": a nucleic acid derived from and specific for *Zea mays* taxon, preferably *Zea mays* ssp. *mays,*
   b) "Bn": a nucleic acid derived from and specific for *Brassica napus* taxon,
   c) "Gm": a nucleic acid derived from and specific for *Glycine max* taxon, and one, more than one or all of nucleic acids chosen from:
   d) "p35S": a nucleic acid derived from the 35S promoter of Cauliflower Mosaic Virus,
   e) "tNOS": a nucleic acid derived from the 3' terminator of the *Agrobacterium tumefaciens* nopaline synthetase gene,
   f) "Cry1Ab": a nucleic acid derived from the crystal protein gene *Cry1Ab* of *Bacillus thuringiensis,*
   g) "PAT/bar": a nucleic acid derived from the phosphinothricin acetyltransferase (PAT) gene *bar* of *Streptomyces hygroscopicus,*
   h) "PAT/pat": a nucleic acid derived from the phosphinothricin acetyltransferase (PAT) gene pat of *Streptomyces viridochromogenes,* and
   i) "CP4-EPSPS": a nucleic acid derived from the 5-Enol-pyruvylshikimate-3-phosphate synthase *EPSPS* gene from *Agrobacterium* sp. CP4; and
(2) concluding the presence or absence in the sample of material derived from one or more transgenic plant events chosen from the group comprising or consisting of: events Bt176, Bt11, Bt10, MON810, MON863, TC1507, NK603, T25, GA21, DAS-59122, crosses thereof, and related events thereof; events Topas 19/2, MS1, RF1, RF2, RF3, MS8, GT73, T45, Liberator pHoe6/Ac, GS40/90pHoe6/Ac, crosses thereof including MS1/RF1, MS1/RF2, MS8/RF3, and related events thereof; events MON 40-3-2, MON89788, A2704-12, A5547-127, crosses thereof, and related events thereof.

Aspect A1 allows to investigate the potential presence or absence in a sample of material from numerous transgenic plant events belonging to several distinct plant taxons, while performing a relatively limited number of detection steps. Advantageously, the transgenic plant events detected by the method of aspect A1 encompass a large fraction, or even substantially most, transgenic plant events presently authorised and/or commercialised, e.g., in Europe, i.e., transgenic plant events which are particularly likely to be encountered in, e.g., environmental or commercial samples. Hence, in this aspect the invention provides a relatively undemanding assay for inspecting a considerable spectrum of relevant transgenic plant events.

Preferably, step (1) of aspect A1 can involve detecting the presence or absence in the sample of nucleic acids comprising or consisting of all nucleic acid listed under a) - i) as defined above. This advantageously improves the information obtained by testing the sample and permits to conclude the potential presence or absence of material derived from a substantially high number of transgenic plant events.

While the method of aspect A1 permits to simultaneously examine the presence or absence in the sample of transgenic plant events belonging to at least three different taxons, the inventors also envisage adapting the method to individual taxons. This may usefully reduce the number of nucleic acid detection steps if information is only required for a particular taxon.

Accordingly, in a further aspect ("A2") the invention provides a method to examine a sample for the presence or absence of material derived from one or more maize transgenic plant events comprising the steps of:
(1) detecting the presence or absence in the sample of nucleic acids comprising or consisting of nucleic acid listed under a) as defined above and one, more than one or all of nucleic acids chosen from those listed under d) - i) as defined above; and
(2) concluding the presence or absence in the sample of material derived from one or more maize transgenic plant events chosen from the group comprising or consisting of events Bt176, Bt11, Bt10, MON810, MON863, TC1507, NK603, T25, GA21, DAS-59122, crosses thereof, and related events thereof.

Preferably, step (1) of aspect A2 can involve detecting the presence or absence in the sample of nucleic acids comprising or consisting of all nucleic acid listed under a) and d) - i) as defined above. This increases the information obtained by testing the sample and permits to conclude the potential presence or absence of material derived from a comparably higher number of maize transgenic plant events.

In a further aspect ("A3") the invention offers a method to examine a sample for the presence or absence of material derived from one or more oilseed rape transgenic plant events comprising the steps of:
(1) detecting the presence or absence in the sample of nucleic acids comprising or consisting of nucleic acid listed under b) as defined above and one, more than one or all of nucleic acids chosen from those listed under d) - i) as defined above; and
(2) concluding the presence or absence in the sample of material derived from one or more oilseed rape transgenic plant events chosen from the group comprising or consisting of events Topas 19/2, MS1, RF1, RF2, RF3, MS8, GT73, T45, Liberator pHoe6/Ac, GS40/90pHoe6/Ac, crosses thereof including MS1/RF1, MS1/RF2, MS8/RF3 and related events thereof.

Preferably, step (1) of aspect A3 can involve detecting the presence or absence in the sample of nucleic acids comprising or consisting of all nucleic acids listed under b) and d) - i) as defined above. This increases the information obtained by testing the sample and permits to conclude the potential presence or absence of material derived from a comparably higher number of oilseed rape transgenic plant events.

In another preferred embodiment, step (1) of aspect A3 involves detecting the presence or absence in the sample of nucleic acids comprising or consisting of nucleic acid listed under b) and d), e), and g) - i) as defined above. It is noted that many oilseed rape events, e.g., those specifically recited in step (2) of aspect A3, may not include nucleic acid listed under f) as defined above. Therefore, omission of the said nucleic acid listed under f) from step (1) of aspect A3 may reduce the number of tests without loss of information.

In a further aspect ("A4") the invention offers a method to examine a sample for the presence or absence of material derived from one or more soybean transgenic plant events comprising the steps of:
(1) detecting the presence or absence in the sample of nucleic acids comprising or consisting of nucleic acid listed under c) as defined above and one, more than one or all of nucleic acids chosen from those listed under d) - i) as defined above; and
(2) concluding the presence or absence in the sample of material derived from one or more soybean transgenic plant events chosen from the group comprising or consisting of events MON 40-3-2, MON89788, A2704-12, A5547-127, crosses thereof, and related events thereof.

Preferably, step (1) of aspect A4 can involve detecting the presence or absence in the sample of nucleic acids comprising or consisting of all nucleic acids listed under c) and d) - i) as defined above. This increases the information obtained by testing the sample and permits to conclude the potential presence or absence of material derived from a comparably higher number of soybean transgenic plant events.

In another preferred embodiment, step (1) of aspect A4 involves detecting the presence or absence in the sample of nucleic acids comprising or consisting of nucleic acid listed under b) and d), e), h) and i) as defined above. It is noted that many soybean events, e.g., those specifically recited in step (2) of aspect A4, may not include nucleic acids listed under f) and g) as defined above. Therefore, omission of the said nucleic acids listed under f) and g) from step (1) of aspect A4 may reduce the number of tests without loss of information.

It shall be also appreciated that further aspects can teach combinations of any two of the above aspects A2 - A4. For example, this may advantageously reduce the number of testing actions in cases where detection of transgenic events from select taxons is intended.

Accordingly, in a further aspect ("A5") the invention offers a method to examine a sample for the presence or absence of material derived from one or more maize and/or oilseed rape transgenic plant events comprising the steps of:
(1) detecting the presence or absence in the sample of nucleic acids comprising or consisting of nucleic acids listed under a) and b) as defined above and one, more than one or all of nucleic acids chosen from those listed under d) - i) as defined above; and
(2) concluding the presence or absence in the sample of material derived from one or more maize and/or oilseed rape transgenic plant events chosen from the group comprising or consisting of events Bt176, Bt11, Bt10, MON810, MON863, TC1507, NK603, T25, GA21, DAS-59122, crosses thereof, and related events thereof; and events Topas 19/2, MS1, RF1, RF2, RF3, MS8, GT73, T45, Liberator pHoe6/Ac, GS40/90pHoe6/Ac, crosses thereof including MS1/RF1, MS1/RF2, MS8/RF3 and related events thereof.

For reasons clarified above, step (1) of aspect A5 can preferably involve detecting the presence or absence in the sample of nucleic acids comprising or consisting of all nucleic acids listed under a), b) and d) - i) as defined above.

In yet a further aspect ("A6") the invention offers a method to examine a sample for the presence or absence of material derived from one or more maize and/or soybean transgenic plant events comprising the steps of:
(1) detecting the presence or absence in the sample of nucleic acids comprising or consisting of nucleic acids listed under a) and c) as defined above and one, more than one or all of nucleic acids chosen from those listed under d) - i) as defined above; and
(2) concluding the presence or absence in the sample of material derived from one or more maize and/or oilseed rape transgenic plant events chosen from the group comprising or consisting of events Bt176, Bt11, Bt10, MON810, MON863, TC1507, NK603, T25, GA21, DAS-59122, crosses thereof, and related events thereof; and events MON 40-3-2, MON89788, A2704-12, A5547-127, crosses thereof, and related events thereof.

For reasons clarified above, step (1) of aspect A6 can preferably involve detecting the presence or absence in the sample of nucleic acids comprising or consisting of all nucleic acids listed under a), c) and d) - i) as defined above.

In yet a further aspect ("A7") the invention offers a method to examine a sample for the presence or absence of material derived from one or more oilseed rape and/or soybean transgenic plant events comprising the steps of:
(1) detecting the presence or absence in the sample of nucleic acids comprising or consisting of nucleic acids listed under b) and c) as defined above and one, more than one or all of nucleic acids chosen from those listed under d) - i) as defined above; and
(2) concluding the presence or absence in the sample of material derived from one or more oilseed rape and/or soybean transgenic plant events chosen from the group comprising or consisting of events Topas 19/2, MS1, RF1, RF2, RF3, MS8, GT73, T45, Liberator pHoe6/Ac, GS40/90pHoe6/Ac, crosses thereof including MS1/RF1, MS1/RF2, MS8/RF3 and related events thereof; and events MON 40-3-2, MON89788, A2704-12, A5547-127, crosses thereof, and related events thereof.

For reasons clarified above, step (1) of aspect A7 can preferably involve detecting the presence or absence in the sample of nucleic acids comprising or consisting of all nucleic acids listed under b), c) and d) - i) as defined above.

In another preferred embodiment, step (1) of aspect A7 involves detecting the presence or absence in the sample of nucleic acids comprising or consisting of all nucleic acids listed under b), c) and d), e) and g) - i) as defined above, i.e., without detecting the nucleic acid listed under f).

As explained, methods of the above aspects A1 - A7 are well-suited for detecting the potential presence or absence in a sample of material derived from relevant transgenic plant events, such as ones that have been authorised and/or are commonly used. However, it shall be appreciated that the said methods are also advantageously flexible and, in embodiments, allow for assessing the presence or absence of material derived from further transgenic events, i.e., events not specifically recited in step (2) of the above aspects A1 - A7. This thus permits to gather even more information about the composition of the sample.

Hence, in embodiments, step (1) of any of the above aspects A1, A2, A5 or A6 may further comprise detecting the presence or absence in the sample of nucleic acid j) "mCry3A": a nucleic acid derived from the modified crystal protein gene *Cry3A* of Bacillus thuringiensis; and the group of events as defined in step (2) of any of the respective aspects further comprises maize event MIR604, crosses thereof with other maize events, and events related to MIR604.

In further embodiments, step (1) of any of the above aspects A1, A2, A5 or A6 may further comprise detecting the presence or absence in the sample of one or both nucleic acids k) "cordapA": a nucleic acid derived from the lysine-insensitive dihydrodipicolinate synthase (cDHDPS) gene *cordapA* of *Corynebacterium glutamicum* and/or I) "Glb1": a nucleic acid derived from the Glb1 promoter of maize; and the group of events as defined in step (2) of any of the respective aspects further comprises maize event LY038, crosses thereof with other maize events, and events related to LY038.

In yet further embodiments, step (1) of any of the above aspects A1, A2, A5 or A6 may further comprise detecting the presence or absence in the sample of nucleic acid m) "Cry3Bb1": a nucleic acid derived from the crystal protein gene *Cry3Bbl* of Bacillus thuringiensis; and the group of events as defined in step (2) of any of the respective aspects further comprises maize event MON88017, crosses thereof with other maize events, and events related to MON88017.

Accordingly, in embodiments, step (1) of any of the above aspects A1, A2, A5 or A6 may further comprise detecting the presence or absence in the sample of one, more than one, or all of nucleic acids listed under j) - m) as defined above; and the group of events as defined in step (2) of any of the respective aspects may further comprise one, more than one, or all of the maize events MIR604, LY038 and MON88017, crosses thereof with other maize events, and events related thereto.

In embodiments, step (1) of any of the above aspects A1, A3, A5 or A7 may further comprise detecting the presence or absence in the sample of nucleic acid n) "Bxn": a nucleic acid derived from the nitrilase gene *Bxn* of *Klebsiella pneumoniae* ssp. *ozaenae;* and the group of events as defined in step (2) of any of the respective aspects further comprises oilseed rape event OXY235, crosses thereof with other oilseed rape events, and events related to OXY235.

In further advantageous embodiments, the methods of the above aspects A1 - A7 can thus additionally also assess the potential presence or absence in samples of material derived from transgenic events that belong to other taxons.

Accordingly, in embodiments, step (1) of any of the above aspects A1 - A7 (or of the above embodiments of said aspects) may further comprise detecting the presence or absence in the sample of nucleic acid o) "Or": a nucleic acid derived from and specific for *Oryza sativa* taxon; and the group of events as defined in step (2) of any of the respective aspects further comprises one, more than one, or all of the rice events LL62, LL06 and LL601, crosses thereof, and events related thereto.

In further embodiments, step (1) of any of the above aspects A1 - A7 (or of the above embodiments of said aspects) further comprises detecting the presence or absence in the sample of nucleic acid p) "Bv": a nucleic acid derived from and specific for *Beta vulgaris* taxon; and the group of events as defined in step (2) of any of the respective aspects further comprises one, more than one, or all of the sugar beet events T120-7, H7-1 and A5-15, crosses thereof, and events related thereto.

In still further embodiments, step (1) of any of the above aspects A1 - A7 (or of the above embodiments of said aspects) further comprises detecting the presence or absence in the sample of nucleic acid q) "Gs": a nucleic acid derived from and specific for *Gossypium* taxon; and the group of events as defined in step (2) of any of the respective aspects further comprises one, more than one, or all of the cotton events LL cotton 25, MON 1445, crosses thereof, and events related thereto.

In a further development, step (1) of any of the embodiments of the preceding paragraph may further comprise detecting the presence or absence in the sample of one or both of nucleic acids r) "Cry1Ac": a nucleic acid derived from the crystal protein gene *Cry1Ac* of *Bacillus thuringiensis* and/or s) "Cry2Ab2": a nucleic acid derived from the crystal protein gene *Cry2Ab2* of *Bacillus thuringiensis;* and the group of events as defined in step (2) further comprises one or both of cotton events MON 531, MON15985, crosses thereof with other cotton events, and events related thereto.

In still further embodiments, step (1) of any of the above aspects A1 - A7 (or of the above embodiments of said aspects) further comprises detecting the presence or absence in the sample of nucleic acid t) "St": a nucleic acid derived from and specific for *Solanum tuberosum* taxon; and the group of events as defined in step (2) of any of the respective aspects further comprises the potato event EH92-527-1 and events related thereto.

In a further development, step (1) of any of the embodiments of the preceding paragraph may further comprise detecting the presence or absence in the sample of nucleic acid u) "GBSS": a nucleic acid derived from the granule bound starch synthase gene *Gbss* of *Solanum tuberosum.*

The invention also teaches an uncomplicated and straightforward way to perform step (2) of the above aspects and embodiments, i.e., to conclude the potential presence or absence in the sample of material derived from the various transgenic plant events of interest. In particular, the results acquired for a sample in step (1) of the above aspects and embodiments are represented as a set "G*_{SAM}*" consisting of, i.e., representing a collection of, those nucleic acids chosen from ones comprising or consisting of those listed under a) - u) above that were detected in the said step (1) as being present in the sample (step (i)).

Moreover, any transgenic plant event of interest can be represented as a set *"*G*ₓ"* (where *X* designates the event of interest), consisting of, i.e., representing a collection of, those nucleic acids chosen from ones comprising or consisting of those listed under a) - u) above that are found in the said event and thus would be detected in step (1) if the sample contained material derived from the said event, or a cross involving such, or a related event (step (ii)).

In particular exemplary embodiments, the set G*ₓ* may preferably be chosen from sets representing the events specifically recited above, e.g., comprising sets: maize event sets *G_{Bt176}* ∈ {Zm; p35S; Cry1Ab; PAT/bar}, *G_{Bt11}* ∈ {Zm; p35S; tNOS; Cry1Ab; PAT/pat}, *G_{Bt10}* ∈ {Zm; p35S; tNOS; Cry1Ab; PAT/pat}, *G_{MON810}* ∈ {Zm; p35S; tNOS; Cry1Ab}, *G_{MON863}* ∈ {Zm; p35S; tNOS}, *G_{TC1507}* ∈ {Zm; p35S; PAT/pat}, *G_{NK603}* ∈ {Zm; p35S; tNOS; CP4-EPSPS}, G_{T25} ∈ {Zm; p35S; PAT/pat}, *G_{GA21}* ∈ {Zm; tNOS}, *G_{DAS-59122}* ∈ {Zm; p35S; PAT/bar}, *G_{MlR604}* ∈ {Zm; tNOS; mCry3A}, *G_{LY038}* ∈ {Zm; p35S; tNOS; cordapA; Glb1}, and *G_{MON88017}* ∈ {Zm; p35S; tNOS; CP4-EPSPS; Cry3Bb1}, oilseed rape event sets *G*_{*Topas* 19/2} ∈ {Bn; p35S; PAT/pat}, *G_{MS1}* ∈ {Bn; tNOS; PAT/bar}, *G_{RF1}* ∈ {Bn; tNOS; PAT/bar}, G_{RF2} ∈ {Bn; tNOS; PAT/bar}, *G*_{*MS1*/*RF1*} ∈ {Bn; tNOS; PAT/bar}, *G*_{*MS1*/*RF2*} ∈ {Bn; tNOS; PAT/bar}, G_{MS8} ∈ {Bn; tNOS; PAT/bar}, *G_{RF3}* ∈ {Bn; tNOS; PAT/bar}, *G_{MS81RF3}* ∈ {Bn; tNOS; PAT/bar}, *G_{GT73}* ∈ {Bn; CP4-EPSPS}, *G_{T45}* ∈ {Bn; p35S; PAT/pat}, *G*_{*LiberatorpHoe6*/*Ac*} ∈ {Bn; p35S; PAT/pat}, *G*_{*GS40*/*90pHoe6*/*Ac*} ∈ {Bn; p35S; PAT/pat}, G*_{OXY235}* ∈ {Bn; p35S; Bxn}; soybean event sets G*_{MON40-3-2}* ∈ {Gm; p35S; tNOS; CP4-EPSPS}, G*_{MON89788}* ∈ {Gm; CP4-EPSPS}, G*_{A2704-12}* ∈ {Gm; p35S; PAT/pat}, and G*_{A5547-127}* ∈ {Gm; p35S; PAT/pat}, rice event sets G*_{LL62}* ∈ {Or; p35S; PAT/bar}, G*_{LL06}* ∈ {Or; p35S; PAT/bar} and G*_{LL601}* ∈ {Or; p35S; tNOS; PAT/bar}, sugar beet event sets G*_{T120-7}* ∈ {Bv; p35S; PAT/pat}, G*_{H7-1}* ∈ {Bv; p35S; CP4-EPSPS}, and G*_{AS-15}* ∈ {Bv; p35S; tNOS; CP4-EPSPS}, cotton event sets G*_{LL cotton 25}* ∈ {Gs; p35S; tNOS; PAT/bar}, G*_{MON1445}* ∈ {Gs; p35S; tNOS; CP4-EPSPSI, G*_{MON531}* ∈ {Gs; p35S; tNOS; cry1Ac} and G*_{MON15985}* ∈ {Gs; p35S; tNOS; cry1Ac; cry2Ab2), and potato event set G*_{EH92-527-1}* ∈ {St; tNOS; Gbss}; wherein the designations between brackets {} refer to those nucleic acids as listed under a) - u) above that are found in such events and may thus be detected in materials derived therefrom. It is to be understood that if the detection step (1) involves additional nucleic acids other than those listed under a) - u) above, such nucleic acids can be advantageously included in the above sets.

Hence, the presence of material derived from an event of interest X in the sample can then be determined by performing for the respective set of interest Gₓ logical operations as follows (step iii):
- if Gₓ equals G*_{SAM}* (Gₓ = G*_{SAM}*), then material derived from the transgenic plant event X, or from a cross thereof, or from an event related thereto, is potentially present in the sample;
- if Gₓ is a proper subset of G_{SAM} (Gₓ c G_{SAM}), then material derived from transgenic plant event X, or from a cross thereof, or from an event related thereto, is potentially present in the sample;
- if Gₓ does not equal G*_{SAM}* and Gₓ is not a proper subset of G*_{SAM}*, (G*ₓ* ≠ G*_{SAM}* and G*ₓ*⊄ G*_{SAM}*), then material derived from transgenic plant event *X,* or from a cross thereof, or from an event related thereto, is absent from the sample.

In preferred embodiments, these logical operations can be advantageously carried out by a computing device.

In particularly preferred embodiments of the above methods, the presence or absence of nucleic acids comprising or consisting of those listed under a) - u) above in a sample is detected using amplification of amplicons comprised in the respective nucleic acids, such as, preferably using PCR and, more preferably using real-time PCR.

Accordingly, in related aspects, the invention also encompasses primers and primer pairs particularly suited for amplification of amplicons within nucleic acids comprising or consisting of those listed under a) - u) above, amplification products so-obtainable using the said primers and primer pairs, vectors and plasmids comprising the said amplification products, and recombinant microorganisms transformed with and harbouring the said vectors and plasmids.

Consequently, in preferred embodiments, the invention also relates to recombinant E. *coli* as deposited under the Budapest Treaty with the Belgian Coordinated Collections of Microorganisms (BCCM), Laboratorium voor Moleculaire Biologie - Plasmidencollectie (BCCM/LMBP) (Universiteit Gent, Technologiepark 927, B-9052 Gent-Zwijnaarde, Belgium) on January 10, 2007 under LMBP accession numbers LMBP 5452, LMBP 5453, LMBP 5454, LMBP 5455, LMBP 5456, LMBP 5457, LMBP 5458, LMBP 5459 and LMBP 5460, on March 6, 2007 under LMBP accession number LMBP 5451, and on April 19, 2007 under LMBP accession numbers LMBP 5587, LMBP 5588, LMBP 5589 and LMBP 5590, as well as to isolated recombinant plasmids obtainable from the said recombinant E. *coli,* as well as to isolated inserts of the said plasmids, preferably isolated *EcoRl-EcoRl* inserts thereof.

Such plasmids or vectors or inserts thereof, and their combinations, are particularly useful as positive controls and/or calibrators for the respective amplicons in the amplification reactions of the above methods.

In addition, the invention also covers kits comprising one or more reagents useful in the methods of one or more of the above aspects and embodiments. For example, the said kits can comprise one or more of the above primers or primer pairs and/or one or more of the above amplification products, vectors or plasmids comprising such, or inserts of the said vectors or plasmids, and/or a data carrier comprising instructions for a programmable computing device to carry out the step (2) of the methods of the invention. Such kits may further conveniently contain instructions for the user to perform the methods of the invention and to interpret the data so-obtained.

These and further aspects and preferred embodiments of the invention are described in the following sections and in the appended claims.

### BRIEF DESCRIPTION OF FIGURES

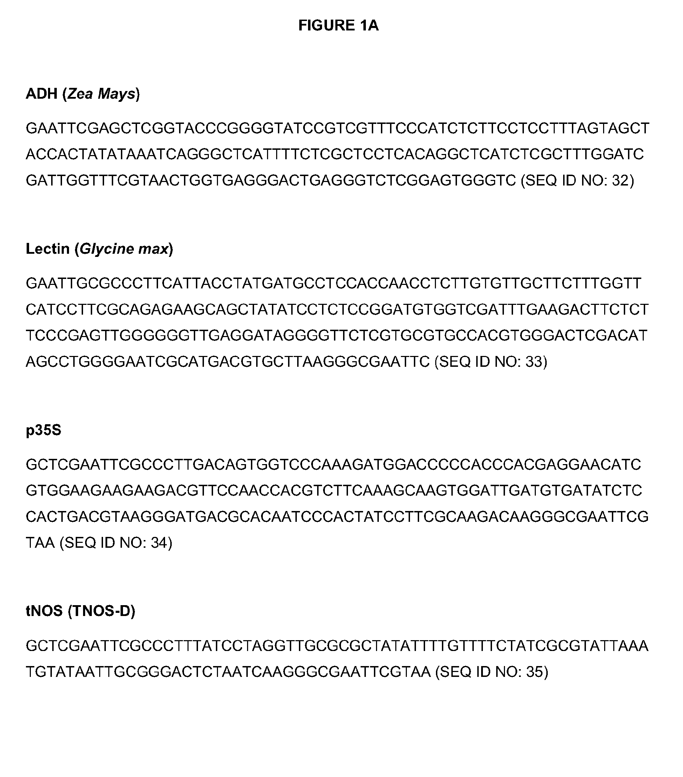

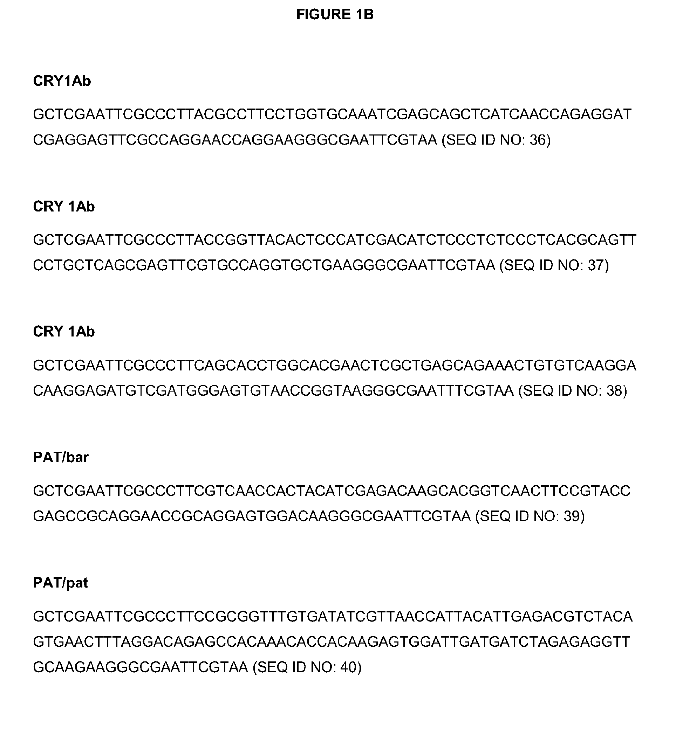

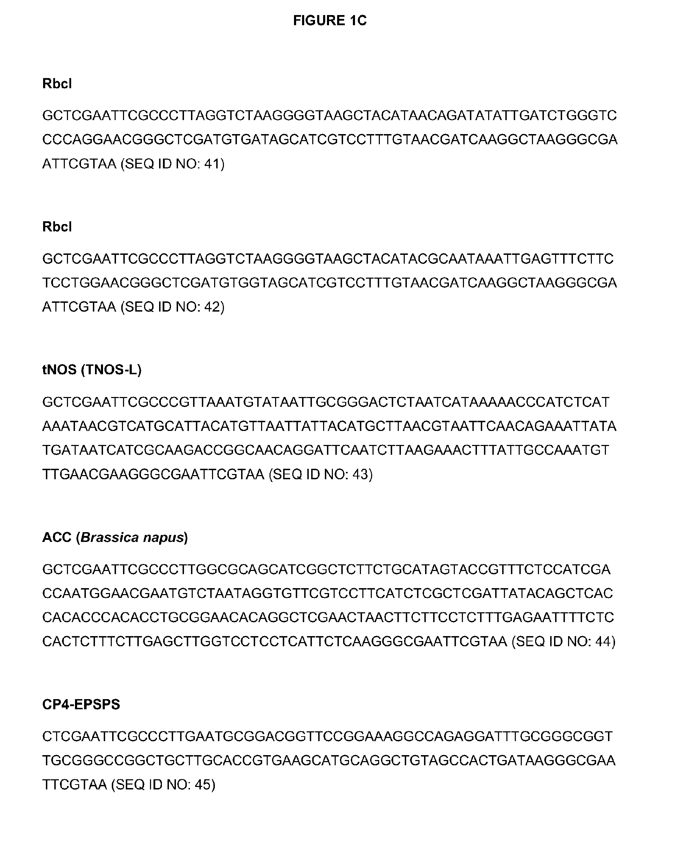

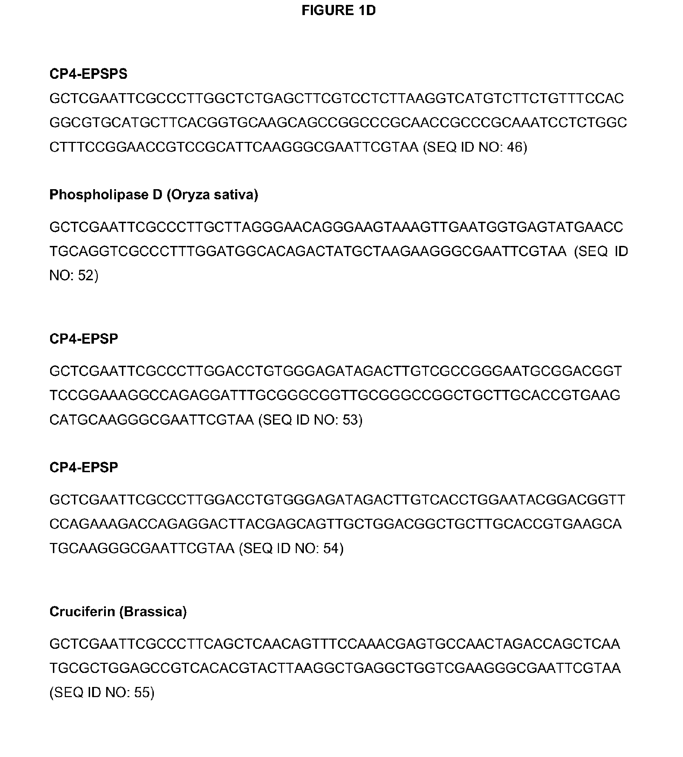

**Figure 1** **(A-D)** illustrates select sequences of amplicons amplified using primers sets listed in Table 3 and present in cloning vectors, particularly in pUC18, such as plasmids listed in Table 4. As can be appreciated, the sequences may include partial sequences from multiple cloning sites flanking the inserted amplicons.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps.

The term "about" as used herein when referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less from the specified value, insofar such variations are appropriate to perform in the disclosed invention.

The recitation of numerical ranges by endpoints includes all numbers and fractions subsumed within that range, as well as the recited endpoints.

All documents cited in the present specification are hereby incorporated by reference in their entirety. In particular, the teachings of all documents herein specifically referred to are incorporated by reference.

Hence, in some aspects, more particularly aspects A1 - A7 as described in the Summary section, the invention concerns methods to examine a sample for the presence or absence of material derived from one or more transgenic plant events comprising the steps (1) detecting the presence or absence in the sample of nucleic acids comprising or consisting of one, more than one or all (the particular choice depending on the goal of a given assay) of nucleic acids listed under a) - u) in the Summary section, and (2) concluding the presence or absence in the sample of material derived from one or more transgenic plant events chosen from the group comprising or consisting of some or all of those detailed in the Summary section.

A "transgenic plant event" occurs with an independent transformation of plant cells with heterologous nucleic acid, typically a nucleic acid construct comprising one or more transgenes of interest; regeneration of a population of plants resulting from an insertion of the construct or a part thereof including the said transgene(s) of interest into the genome of the plants; and selection of a particular plant characterised by the said insertion into one or more, preferably one, particular genome location. The term "transgenic plant event" thus encompasses the original so-selected transformant plant, as well as any successive progeny thereof comprising the inserted nucleic acid including the transgene(s) of interest; for example, the said progeny may be hemizygous or homozygous for the said insertion; the said progeny may be obtained, e.g., by vegetative propagation or by sexual crossing.

The term "plant" as used herein generally encompasses plant cells; plant protoplasts; plant tissues; plant cells or tissue culture from which a plant organism can be regenerated; plant calli or clumps; plant organisms and parts thereof, such as, without limitation, flowers, tepals, petals, sepals, anthers, pollen, seeds, fruit, pericarp, pods, leaves, petioles, stems, roots, rhizomes, stolons, tubers, shoots, and the like. The term as used herein generally encompasses plants of any taxons classified in the art within the kingdom Plantae. In addition, plant as understood herein may preferably belong to land plants (embryophytes) including, without limitation, non-vascular plants (bryophytes) and vascular plants (tracheophytes); more preferably, to vascular plants (tracheophytes) including, without limitation, lycopodiophyta, equisetophyta, pteridophyta, psilotophyta, ophioglossophyta, and seed plants (spermatophytes); even more preferably to seed plants (spermatophytes) including, without limitation, seed-bearing plants (gymnosperms) such as, e.g., pinophyta, cycadophyta, ginkgophyta and gnetophyta and flowering plants (angiosperms) such as magnoliophyta, including monocots (liliopsida) and dicots (Magnoliopsida). Preferred plants may be ones commonly applied in agriculture or horticulture. By means of example and not limitation, preferred crop plants can include maize, wheat, rice, barley, sorghum, tobacco, tomato, potato, oilseed rape (rapeseed), soybean, sugar beet, pea, sunflower, cotton, peanut, flowers (e.g., carnation), etc.

The term "material" generally refers to physical matter. The term "material derived from a transgenic plant event" encompasses the transgenic plant event as such, including, by means of example and not limitation, the transgenic plant event organism; any parts of the transgenic plant event organism, such as, e.g., flowers, tepals, petals, sepals, anthers, pollen, seeds, fruits, pericarp, pods, leaves, petioles, stems, roots, rhizomes, stolons, tubers or shoots, or portions thereof; cells, protoplasts, tissues, calli or clumps of the transgenic plant event; or material obtained by subjecting any of the above to one or more downstream processing steps. These downstream processing steps may comprise any actions used to process particular plant materials in agriculture, horticulture or in any downstream industries, e.g., food industry including beverage industry, feed industry, textile industry (e.g., cotton, linen, bamboo, etc.), fuel industry (e.g., oilseed rape), lubricant industry (e.g., castor oil), paint industry (e.g., linseed oil, tung oil), cosmetic and pharmaceutical industry, etc.

By means of example and not limitation, such downstream processing may include harvesting; removal of unwanted outer layers, e.g., peeling, skinning, etc.; drying, e.g., air drying, sun drying, spray drying, freeze drying, juice concentrating, etc.; diminution, e.g., grinding, milling, chopping, slicing, etc.; liquefaction, e.g., juice collection; preserving, e.g., vacuum bottling, tinning, canning, pasteurization, addition of preservatives, etc.; pressing, e.g., oil collection; hydrogenation, e.g., vegetable oil saturation; macerating; emulsifying; heat treatment, e.g., cooking, pressure cooking, steaming, oven baking, smoking, frying, grilling, etc.; fermenting, e.g., by yeast, bacteria and/or fungi; freezing; and the like.

The term "sample" as used herein refers broadly to a representative part or fraction of a larger material whole being presented for inspection, such as, e.g., for quality control. Preferably, a sample to be examined by methods of the invention may be suspected of potentially comprising material derived from one or more transgenic events of interest. By means of example, such suspicion may exist for any sample representative of material that is known as comprising, or suspected of probably or possibly comprising, plants or parts thereof or material derived therefrom. In a preferred example, the sample may be representative of material that is known as comprising, or suspected of probably or possibly comprising, plants or parts thereof or material derived therefrom, wherein the said plants or parts thereof belong to the same taxon as a transgenic event of interest whose presence in the sample is to be examined. For example, a sample may be representative of plants or parts thereof, e.g., collected or harvested plants or parts thereof (such as, without limitation, fruits, seeds, pods, flowers, etc.), or representative of an intermediary or final material obtained by applying one or more downstream processing steps thereto, or of products comprising such material, e.g., of processed food or feed products.

Hence, in an embodiment the sample can comprise plants or parts thereof, including flowers, tepals, petals, sepals, anthers, pollen, seeds, fruits, pericarp, pods, leaves, petioles, stems, roots, rhizomes, stolons, tubers or shoots, or portions thereof, plant cells, plant protoplasts and/or plant tissues, and/or plant-derived material, preferably food or feed material, including processed food or feed material.

The methods of the invention detect the presence or absence in the sample of nucleic acids, i.e., of genetic material and preferably genomic DNA, originating or derived from transgenic plant events of interest. The presence or absence of such nucleic acids is indicatory of, respectively, the presence or absence in the sample of this and potentially further material derived from the respective transgenic plant events; such as, e.g., materials co-purifying or co-separating with the said nucleic acids during the various processing steps applied to the transgenic plant events.

It is known that nucleic acids, and particularly genomic DNA, are comparably durable and can tolerate a variety of processing steps applied to plant materials in agriculture and industry, e.g., in food or feed industry, such that DNA molecules of lengths that permit sequence-specific detection thereof may be found following such steps and even in final products.

Accordingly, a preferred sample of the invention comprises nucleic acids, more preferably genomic DNA, even more preferably genomic DNA having at least sizes that allow for sequence specific-detection thereof, such as, e.g., on average, at least about 20 bp, e.g., at least about 30 bp, preferably at least about 50 bp, e.g., at least about 75 bp, more preferably at least about 100 bp, e.g. about 150 bp, or even more preferably at least about 200 bp, or at least about 300 bp, or at least about 500 bp, or at least about 1 kb, or more.

Although it can be assumed that most processing steps to which a transgenic plant event would be commonly exposed in practice would preserve detectable nucleic acids as above in the eventual product, and thus in the sample thereof to be examined, the inventors also envisage a positive control aimed at verifying whether the said sample comprises detectable plant-derived nucleic acids.

In particular, the method of the invention may further comprise detecting the presence or absence in the sample of a generic plant-derived nucleic acid. In a preferred embodiment, such generic plant-derived nucleic acid is derived from a gene present in and preferably highly conserved (e.g., ≥ 80%, preferably ≥ 90%, more preferably ≥ 95%, even more preferably ≥ 96%, ≥ 97%, ≥ 98% or ≥ 99% sequence identity) between various plant taxons; preferably at least within spermatophytes; or at least within angiosperms, such as between and/or within monocots and dicots; or preferably at least between plant taxons commonly used in agriculture and industry, such as, without limitation, between maize, wheat, rice, barley, sorghum, tobacco, tomato, potato, oilseed rape (rapeseed), soybean, sugar beet, pea, sunflower, cotton, peanut and flowers (e.g., carnation); or preferably at least between plant taxons comprising or consisting of those of the transgenic plant events tested in the sample.

In preferred embodiments, the said generic plant-derived nucleic acid is derived from the chloroplastic small subunit of Rubisco gene or from the CHL-tRNA synthetase gene.

As mentioned, the methods of the invention include in step (1) detecting the presence or absence in the sample of nucleic acids comprising or consisting of one, more than one or all of those listed under a) - u) in the Summary section. The term "detecting" or "detection" as used herein encompasses any means of determining the presence or absence of a given nucleic acid in the sample.

Preferably, the presence of a particular nucleic acid molecule in a sample can be detected using one or more reagents specifically hybridising to respective nucleotide sequence(s) comprised within the said nucleic acid molecule.

The terms "hybridisation" and "hybridise" refer to a process by which a nucleic acid strand anneals with complementary or substantially complementary sequence(s) comprised in the same or another polynucleotide strand through base pairing, preferably Watson-Crick base pairing.

The terms "complementary" and "complementarity" refer to the normal binding of polynucleotides under permissive salt (ionic strength) and temperature conditions by base pairing, preferably Watson-Crick base pairing. By means of example, complementary Watson-Crick base pairing occurs between the bases A and T, A and U or G and C. For example, the sequence A-G-T (i.e., 5'-A-G-T-3') is thus complementary sequence A-C-T (i.e., 5'-A-C-T-3').

The "degree of complementarity" of a nucleic acid strand (A) to a nucleic acid strand (B) can be expressed as the proportion (percentage) of nucleotides of the nucleic acid strand (A) that would be expected to match, i.e., form Watson-Crick base-pairing, with nucleotides of the nucleic acid strand (B), when the said nucleic acid strands (A) and (B) were annealed, preferably in high stringency conditions.

"Complementary" as used herein thus refers to wholly complementary, such that all respective nucleotides of the nucleic acid strands would bind when the strands anneal. By means of example, a relatively shorter nucleic acid strand would show total complementarity to a relatively longer nucleic acid strand, if the latter strand comprised a sequence fully complementary to the sequence of the former strand. "Substantially complementary" refers to largely but not wholly complementary, in particular, to at least 85% complementary, e.g., at least 90% complementary, preferably at least 95% complementary, e.g., at least 96%, 97%, 98% or at least 99% complementary.

"Specifically hybridise" and "specific hybridisation" reflects a situation when a reagent hybridises to a given sequence more readily than it would hybridise to a random, unrelated sequence. For example, a reagent specifically hybridising to a given nucleotide sequence (A) preferably displays little or no hybridisation to other polynucleotides, and preferably to homologues or orthologues of the nucleic acids sequence (A), under conditions where it would specifically hybridise with the said polynucleotide (A).

Preferably, reagents that can specifically hybridise to the respective nucleotide sequence(s) comprised within the nucleic acid molecules listed under a) - u) above can be probes or primers.

A "probe" is an isolated nucleic acid to which is desirably attached a detectable label or reporter moiety, e.g., a radioactive isotope (e.g., ³²P, ³³P), ligand, chemiluminescent agent, fluorophore (e.g., fluorescein, tetrachloro-fluorescein, TAMRA, ROX, Cy3, Cy3.5, Cy5, Cy5.5, Texas Red, etc.), vitamin (e.g., biotin), steroid (e.g., digoxin), enzyme (e.g., HRP, AP, etc.), etc. Such a probe is complementary or substantially complementary to a sequence comprised in a strand of a target nucleic acid, in the case of the present invention, of a nucleic acid, preferably genomic DNA, listed under a) - u) above. Probes according to the present invention may be deoxyribonucleic acids, ribonucleic acids, or nucleic acids comprising both deoxyribo- and ribonucleotides; may comprise standard purine and/or pyrimidine bases (A, G, T, C, U and/or I), but also other natural, chemically or biochemically modified (e.g., methylated), non-natural, or derivatised nucleotide bases; may include backbone comprising sugars and phosphate groups, as can typically be found in RNA or DNA, as well as one or more modified or substituted (such as, 2'-O-alkylated, e.g., 2'-O-methylated or 2'-O-ethylated; or 2'-O,4'-C-alkynelated, e.g., 2'-O,4'-C-ethylated) sugars or one or more modified or substituted phosphate groups - for example, backbone analogues in nucleic acids may include phosphodiester, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs).

Probes may be at least 10 nucleotides in length, e.g., at least 11, at least 12, at least 13 or at least 14 nucleotides in length, preferably at least 15 nucleotides, e.g., at least 16, at least 17, at least 18 or at least 19 nucleotides in length, more preferably at least 20 nucleotides in length, e.g., at least 21, at least 22, at least 23, or at least 24 nucleotides in length, even more preferably at least 25 nucleotides in length, e.g., at least 30, at least 35, at least 40, at least 45, at least 50, at least 75, at least 100 or at least 200 or more nucleotides in length.

"Primers" are isolated nucleic acids, preferably deoxyribonucleic acids or, in other preferred examples, ribonucleic acids or PNAS, complementary or substantially complementary to a sequence comprised in a target nucleic acid (in the case of the present invention, to a sequence comprised in a nucleic acid, preferably genomic DNA, comprising or consisting of those listed under a) - u) above), that can be annealed to the said target nucleic acid and can act as a point of initiation of synthesis of a primer extension product in the presence of nucleotides and an agent for nucleic acid polymerization, such as DNA dependent or RNA dependent polymerase.

A primer needs to be sufficiently long to prime the synthesis of an extension products in the presence of an agent for polymerization. A typical primer may thus be at least 10 nucleotides in length, e.g., at least 11, at least 12, at least 13 or at least 14 nucleotides in length, preferably at least 15 nucleotides in length, e.g., at least 16, at least 17, at least 18 or at least 19 nucleotides in length, more preferably at least 20 nucleotides in length. Further preferred primers are between about 10 and about 40 nucleotides in length, more preferably between about 15 and about 30 nucleotides in length, most preferably between about 20 and about 25 nucleotides long.

"Primer pairs" refer to combinations of primers which are suited for amplification of amplicons from within target nucleic acids (in the case of the present invention, from within nucleic acids, preferably genomic DNA, comprising or consisting of those listed under a) - u) above by suitable nucleic-acid amplification methods. Accordingly, the ability to amplify an amplicon from within a given target nucleic acid using a primer pair designed to specifically hybridise within the said target nucleic acid indicates the presence (and optionally quantity) of the said target nucleic acid in the sample.

Exemplary but non-limiting methods for preparing and using probes and primers are described, for example, in Molecular Cloning: A Laboratory Manual, 2nd ed., vol. 1-3, ed. Sambrook el al., Cold Spring Harbor Laboratory Press, Cold Spring Harbor, N.Y., 1989 ("Sambrook et al. 1989"); Current Protocols in Molecular Biology, ed. Ausubel et al., Greene Publishing and Wiley-Interscience, New York, 1992 (with periodic updates) ("Ausubel et al. 1992"); and Innis et al., PCR Protocols: A Guide to Methods and Applications, Academic Press: San Diego, 1990. PCR-primer pairs can be derived from a known sequence, for example, by using computer programs intended for that purpose such as Primer3 (Rozen and Skaletsky. 2000. Primer3 on the WWW for general users and for biologist programmers. In: Krawetz S, Misener S (eds) Bioinformatics Methods and Protocols: Methods in Molecular Biology. Humana Press, Totowa, NJ, pp 365-386) or the GCG™ v. 11.1.2 package from Accelrys.

Specific hybridisation of the probes, primers or primer pairs of the invention to their respective complementary sequences within the target nucleic acids, e.g., those comprising or consisting of those listed under a) - u) above, can be preferably achieved under high stringency hybridisation conditions.

"High stringency" conditions include conditions equivalent to the following exemplary conditions for binding or hybridisation at 65 °C in a solution consisting of 5xSSPE (43.8 g/I NaCl, 6.9 g/l NaH₂PO₄.H₂O and 1.85 g/l EDTA, pH adjusted to 7.4 with NaOH), 0.1% SDS, 5xDenhardt's reagent (50xDenhardt's contains per 500 ml: 5 g Ficoll (Type 400, Pharmacia), 5 g BSA (Fraction V; Sigma) and 100 µg/ml denatured salmon sperm DNA), followed by washing in a solution comprising 5×SSPE, 0.1 % SDS at 65 °C when a probe of about 500 nucleotides in length is employed. Other exemplary conditions for hybridisation at "high stringency" for nucleic acid sequences over approximately 20-100 nucleotides in length, preferably about 30-100 nucleotides in length, e.g., between 30-50 nucleotides in length, include conditions equivalent to hybridisation in 6×SSC at 45°C, followed by one or more washes in 0.2xSSC, 0.1 % SDS or even 0.1×SSC, 0.1 % SDS at 65°C. Numerous equivalent conditions may be employed to vary stringency conditions; factors such as the length and nature (DNA, RNA, base composition) of the probe and nature of the target (DNA, RNA, base composition, present in solution or immobilised, etc.) and the concentration of the salts and other components (e.g., the presence or absence of formamide, dextran sulphate, polyethylene glycol) are considered and the hybridisation solution may be varied to generate conditions of low or high stringency hybridisation different from, but equivalent to, the above listed conditions. In addition, the art knows conditions that promote hybridisation under conditions of high stringency (e.g., increasing the temperature of the hybridisation and/or wash steps, the use of formamide in the hybridisation solution, etc.). Guidance for performing hybridisation reactions can be found, for example, in Current Protocols in Molecular Biology, John Wiley & Sons, N.Y., 6.3.1-6.3.6, 1989, and more recent updated editions, all of which are incorporated by reference.

Regarding the amplification of amplicons from within the target nucleic acids using primer pairs, "stringent conditions" or "high stringency conditions" are conditions that permit a primer pair to hybridise only to its respective target nucleic-acid sequence and to produce a unique amplification product, i.e., the amplicon, substantially without producing non-intended, i.e., non-specific amplification products. For example, preferably less than 20%, more preferably less than 10%, even more preferably less than 5%, e.g., less than 4%, less than 3%, or less than 2%, yet more preferably less than 1 %, e.g., less than 0.1 % or less than 0.01 % of the total quantity (e.g., weight) of amplification product in an amplification reaction would be non-specific when such reaction is performed at stringent conditions (e.g., as analysed by quantitative gel electrophoresis, or by Tm determination, or the like).

Any conventional methods can be used to detect specific hybridisation of a probe to a target nucleic acid in the sample. For example, nucleic acids, preferably DNA, from a sample may be immobilised and denatured on a solid support and so-hybridised with respective probes (e.g., Southern blotting, slot blotting, dot blotting, etc.). If a probe - and consequently its detectable label - is retained on the solid support, this indicates that the target nucleic for which the probe is specific is present in the sample.

Similarly, any existing nucleic-acid amplification method can be used to amplify amplicons from within target sequences of interest, thereby indicating the presence of the corresponding target sequences in the sample: including but not limited to the polymerase chain reaction (PCR) (US 4,683,202; US 4,965,188), strand displacement amplification (SDA) (US 5,455,166; EP 0684315), LCR, TAS, 3SR, NASBA (US 5,409,818; EP 0329822), RCA and Q-beta amplification.

In a particularly preferred embodiment, the methods of the invention employ polymerase-chain reaction (PCR) to amplify amplicons from within target nucleic acids chosen from ones comprising or consisting of those listed under a) - u) above. The terms "polymerase-chain reaction" and "PCR" broadly covers any method referred as such in the art and in particular methods for amplification of target nucleic acid sequences, especially target DNA sequences, using heat-stable DNA polymerase(s) and two primers, especially oligonucleotide primers, one complementary to the (+)-strand at one end of the sequence to be amplified and the other complementary to the (-)-strand at the other end. The term encompasses modifications of the prototypic PCR, such as, e.g., high-fidelity PCR, hot-start PCR, touch-down PCR, nested PCR, multiplex PCR, quantitative PCR, quantitative real-time PCR, long-range PCR, RT-PCR, etc. (see, e.g., PCR Protocols: A Guide to Methods and Applications, eds. Innis et al., Academic Press, San Diego, 1990).

Amplification products obtained using the above amplification methods, and in particular using PCR, can be evaluated, e.g., for their presence or absence, for their specificity and/or quantity, by any of the manners common in the art. For example, the size (as an indication of amplification specificity) and/or quantity of an amplification product may be evaluated using gel electrophoresis, such as, e.g., agarose or polyacrylamide gel electrophoresis, where amplification products are visualised using suitable DNA-binding dyes, such as, e.g., ethidium bromide.

Alternatively, amplification products may be evaluated by methods that employ probes hybridising to specific sequences within the amplification products. For instance, an amplification product may be immobilised and denatured on a solid support and subsequently hybridised with a labelled probe.

Otherwise, the amplification product may itself be labelled, e.g., by including a detectable label (e.g., a fluorophore) in one or both primers and/or by virtue of substrate nucleotides incorporated into the amplification product during amplification, and the so-obtained PCR product may be subsequently denatured and hybridised with specific (oligonucleotide) probes attached to a solid support. The presence and quantity of the detectable label in select positions on the solid support thus indicates the specificity and quantity, respectively, of the amplification product. For instance, such set-up is suitable for use with probe arrays, e.g., microarrays.

In further exemplary manners, amplification products may be evaluated using cloning and sequencing; direct sequencing; oligonucleotide-mediated pyrosequencing (Ahmadian et al. 2000. Anal Biochem 280: 103-110); chromatography, e.g., DHPLC, oligonucleotide ligation assays (Landegren et al. 1988. Science 241: 1077; Eggerding et al. 1995. Hum Mutat 5: 153-165; Nickerson et al. 1990. PNAS 87: 8923-8927); RNAse Protection Assay; etc.

A particularly advantageous manner of evaluating the amplification product is real-time amplification, especially real-time PCR. The term "real-time amplification" is intended to mean any amplification technique, preferably PCR ("real-time PCR"), which makes it possible to monitor the evolution of an ongoing amplification reaction (see, e.g., Real-Time PCR: An Essential Guide, eds. Edwards et al., Horizon Scientific Press, 2004; Marras SAE et al. 2006. Real-time assays with molecular beacons and other fluorescent nucleic acid hybridization probes. Clin Chim Acta 363: 48-60; for discussion of various real-time PCR platforms).

By means of example and not limitation, real-time amplification, especially real-time PCR, as intended herein encompasses fully conventional systems, such as, e.g., the TaqMan ^{™} system developed by Applied Biosystems, which relies on the release and detection of a fluorogenic probe during each round of DNA amplification (Holland et al. 1991. Detection of specific polymerase chain reaction product by utilizing the 5'-3' exonuclease activity of Thermus aquaticus DNA polymerase. PNAS 88: 7276-80). The method uses the 5' exonuclease activity of Taq polymerase during primer extension to cleave a dual-labelled, fluorogenic probe hybridised to the target DNA between the PCR primers. Prior to cleavage, a reporter fluorophore, such as 6-carboxyfluorescein (6-FAM) at the 5' end of the probe is quenched by 6-carboxy-tetramethylrhodaniine (TAMRA) through fluorescent resonance energy transfer (FRET). Following digestion, FAM is released. The resulting fluorescence measured in real-time at around 518 nm during the log phase of product accumulation is proportional to the number of copies of the target sequence.

Further real-time amplification, especially real-time PCR, detection systems can also utilise FRET, such as, e.g., systems based on molecular beacons. Molecular beacons are single-stranded polynucleotide probes that possess a stem-and-loop hairpin structure. The loop portion is a probe sequence complementary to a sequence within an amplicon to be evaluated, and the stem is formed by short complementary sequences located at the opposite ends of the molecular beacon. The molecular beacon is labelled with a fluorophore (e.g., 6-FAM) at one end and a quencher (e.g., TAMRA) at the other end. When free in solution, the stem keeps the fluorophore and the quencher in close proximity, causing the fluorescence of the fluorophore to be quenched by FRET. However, when bound to its complementary target, the probe-target hybrid forces the stem to unwind, separating the fluorophore from the quencher, and restoring the fluorescence. Accordingly, when the quantity of an amplicon increases during amplification, this can be monitored as an increase in the fluorescence of the corresponding beacon (see, e.g., Manganelli et al. 2001. Real-time PCR using molecular beacons. Methods Mol Med 54: 295-310; Marras SAE. 2006. Selection of fluorophore and quencher pairs for fluorescent nucleic acid hybridization probes. Methods Mol Biol 335: 3-16; Marras SAE et al. 2006. Real-time assays with molecular beacons and other fluorescent nucleic acid hybridization probes. Clin Chim Acta 363: 48-60 for further discussion of molecular beacons detection).

For description of additional ways to detect and evaluate amplification products in real-time (e.g., using adjacent probes; 5'-nuclease probes such as Taqman^{™}; Light-up probes; Duplex scorpion primers; Amplifluor primers; and further alternative fluorescent hybridisation probe formats see, e.g., Marras SAE et al. 2006. Real-time assays with molecular beacons and other fluorescent nucleic acid hybridization probes. Clin Chim Acta 363: 48-60, esp. section 6 and references therein).

In preferred embodiments, the real-time amplification, preferably real-time PCR, of the invention uses (substantially) sequence non-specific reagents to detect and evaluate the accumulation of amplification products. These systems usually employ fluorochromes (fluorescent dyes) that bind to DNA (i.e., DNA-binding), preferably to double-stranded DNA, in a substantially sequence non-specific way, and upon such binding their fluorescence is attained or enhanced. Accordingly, the observed increase in fluorescence is indicative of the appearance and quantity of the accumulated (double-stranded) amplification product. If the said fluorochromes preferentially or exclusively bind to double-stranded DNA and/or if their fluorescence is preferentially or exclusively enhanced when bound to double-stranded DNA, then the specificity of the amplification product can be determined by performing its "melting curve", i.e., fluorescence vs. temperature plot, and determining the Tm of the product, i.e., temperature at which 50% of the product is melted and has thus lost its enhanced fluorescence. The observation of a single expected Tm for an amplification product (or multiple expected Tm in multiplex amplification) corroborates the specificity of amplification.

Exemplary fluorochromes for use in the above sequence non-specific detection methods include, without limitation, major-groove binders, minor-groove binders, intercalating dyes, or the like; such as, e.g., SYBR Green I (2-[N-(3-dimethylaminopropyl)-N-propylamino]-4-[2,3-dihydro-3-methyl-(benzo-1,3-thiazol-2-yl)-methylidene]-1-phenyl-quinolinium; Zipper et al. 2004. Nucleic Acid Res 32: e103), ethidium bromide, Hoechst 33258, PicoGreen™ (Molecular Probes), preferably SYBR Green I or PicoGreen™.

Hence, target for the above described detection using probes and/or template for the amplification using primer pairs, would be nucleic acid originating from respective transgenic plant events comprised in the sample. Preferably, this target and/or template nucleic acid would be genomic DNA originating from the said transgenic events which, due to its greater stability, is expected to be better preserved in the sample than other nucleic acid types, such as, e.g., hnRNA or mRNA. Nevertheless, the invention also contemplates the detection of mRNA or cDNA (e.g., by corresponding RT-PCT assays), or plasmid-derived DNA, where applicable.

The present method thus preferably employs amplification using primers pairs designed to specifically hybridise within and amplify corresponding amplicons from within nucleic acids the presence or absence of which in the sample need to be detected, such as, preferably, nucleic acids comprising or consisting of those listed under a) - u) above, as a means to detect the presence or absence of the said nucleic acids in the sample.

In this respect, the present inventors realised that if material of a transgenic plant event has been exposed to one or more downstream processing steps, e.g., as described above, the nucleic acids, such as the genomic DNA thereof, may be fragmented. Therefore, to increase the chances of the respective amplicons to be representatively amplified from so-fragmented DNA, the invention also teaches to advantageously reduce the size of the amplicons.

Accordingly, in preferred embodiments, the amplicons are less than about 300 bp, e.g., less than 280 bp, less than 260 bp, less than 240 bp, or less than 220 bp, more preferably less than about 200 bp, e.g., less than 190 bp, less than 180 bp, less than 170 bp or less than 160 bp, even more preferably less than about 150 bp, e.g., less than 140 bp, less than 130 bp, less than 120 bp or less than 110 bp, yet more preferably less than about 100 bp, and still more preferably less than about 90 bp, e.g. about 85 bp, about 80 bp, about 75 bp, about 70 bp, about 65 bp, about 60 bp, about 55 bp or about 50 bp.

In further preferred embodiments, the amplicons are between about 50 bp and about 150 bp, between about 50 bp and about 100 bp, preferably between about 50 bp and about 90 bp, more preferably between about 60 bp and about 80 bp, and yet more preferably between about 65 bp and about 75 bp.

As explained, the above methods detect particular nucleic acids, preferably genomic DNA, in a sample. It can be appreciated that some robust detection protocols, e.g., some PCR protocols, might be performed on samples without prior purification or enrichment of their constituent nucleic acids. On the other hand, the detections of the invention may be performed more consistently if nucleic acids, such as preferably genomic DNA, are first enriched or isolated from the sample. Methods for isolation of nucleic acids, such as genomic DNA, from samples, particularly samples comprising plant material, are long-established in the art and include methods based on, by example and not limitation, organic solvent extraction (e.g., phenol-chloroform extraction) and ethanol or isopropyl alcohol precipitation; binding to ion-exchange resins; caesium chloride density separation; spin-column chromatography, magnetic separation, etc. (see, e.g., Milligan 1992. Plant DNA isolation. pp 59-88 in Hoelzel, ed. Molecular genetic analysis of populations: a practical approach. IRL Press, Oxford, UK).

Quality of so-isolated DNA can be assessed, e.g., by gel electrophoresis and/or by measuring the A₂₆₀/A₂₈₀ absorbance ratio. Preferably, the A₂₆₀/A₂₈₀ ratio for nucleic acids used in the detection methods of the invention can be between 1.6 and 2.3, more preferably between 1.6 and 2.0, even more preferably between 1.7 and 1.9, yet more preferably between 1.75 and 1.85 and most preferably about 1.8. Quantity of so-isolated DNA may be assessed, e.g., by measuring absorbance A₂₆₀ (Sambrook 1989), or preferably using PicoGreen™ or SYBR Green I fluorochromes (Ahn et al. 1996. Nucleic Acids Res 24: 2623-5; Schneeberger et al. 1995. PCR Methods Appl 4: 234-8).

In a preferred embodiment, preparation of nucleic acid material, particularly genomic DNA, for use in the methods of the invention may also entail fragmentation to obtain lower average sizes of the nucleic acid fragments, such as, e.g., using enzymatic digestion or sonication as known in the art. Preferable average length of the so-fragmented nucleic acids may be between 200 and 2000 bp, more preferably between 300 and 1500 bp, even more preferably between 500 and 1000 bp, e.g., about 500, about 600, about 700, about 800 about 900 or about 1000 bp.

The above methods thus detect the presence or absence of select nucleic acids in a sample, preferably using detection systems and protocols as described heretofore. As used herein, the "presence" of a given nucleic acid in a sample is generally concluded when the signal intensity value (signal quantity) obtained for that nucleic acid using a suitable detection assay (e.g., fluorescent signal, chemiluminiscent signal, enzymatic reaction signal, etc.) in the sample is greater, preferably statistically significantly greater, than that in a negative control.

A negative control does not contain the nucleic acid being assayed, but can advantageously be comparable to the sample in other respects, preferably most other respects, e.g., in the type and quantity of plant material contained therein, in downstream processing steps to which the material has been exposed, etc.

Preferably, the detection methods of the invention may be performed on nucleic acids, more preferably on genomic DNA, isolated from the sample (see above). In such instance, the negative control may consist essentially of isolated nucleic acids, not comprising the nucleic acid subject to detection. Preferably, the detection method would then employ the same or about the same quantity of isolated nucleic acids from both the sample and, for reference, the negative control. Also preferably, the purity and/or quality of nucleic acids isolated from the sample and from the negative control may also be the same or about the same; further preferably, the same isolation method may be used for isolating nucleic acids from both the sample and the negative control.

In the above situations, the presence of a given nucleic acid in a sample may be preferably concluded when the signal intensity value (signal quantity) obtained for that nucleic acid in the sample is at least 2 × greater than that in the negative control, e.g., at least 3 × or at least 4 × greater, more preferably at least 5 × greater, e.g., at least 6 × greater, at least 7 × greater, at least 8 × greater or at least 9 × greater, even more preferably at least 10 × greater, e.g., at least 15 × greater, yet more preferably at least 20 × greater, e.g., at least 30 × greater or at least 40 × greater, still more preferably at least 50 × greater, e.g., at least 100 × greater, at least 200 × greater, at least 300 × greater, at least 400 × greater, at least 500 × greater, at least 1000 × greater or even greater than the signal in the negative control.

Hence, when the signal value (signal quantity) obtained for the assayed nucleic acid in the sample is not greater (e.g., is same, about same or lower) than the signal value obtained for the assayed nucleic acid in the negative control, or preferably is not greater by the above recited preferred multiples, the "absence" of the said nucleic acid in the sample can be generally concluded.

In preferred embodiments, the presence or absence in the sample of a nucleic acid of interest is evaluated by real-time amplification, preferably real-time PCR. In these systems, a common measure used to express the quantity of the template for a given nucleic acid in a sample is the *Cₜ* value. Briefly, the *Cₜ* value states the cycle number at which the fluorescence attributable to the accruing amplification product (ΔRₙ) passes through an arbitrary threshold fluorescence value, the latter set above the baseline fluorescence but within the exponential phase of amplification (i.e., within the portion which would appear linear on a log 2 plot of the fluorescence vs. cycle # data).

Accordingly, in real-time amplification, the presence of a given nucleic acid in a sample may be generally concluded when *Cₜ* of the sample ("SAM *Cₜ"*) is lower than *Cₜ* of a negative control ("NC *Cₜ"*)*;* and may be preferably concluded when SAM *Cₜ* ≤ (NC *Cₜ -* 1)*,* e.g., SAM *Cₜ* ≤ (NC Ct - 2), SAM *Cₜ* ≤ (NC *Cₜ* - 3) or SAM *Cₜ* ≤ (NC *Cₜ* - 4), more preferably when SAM *Cₜ* ≤ (NC *Cₜ* - 5), e.g., SAM *Cₜ* ≤ (NC *Cₜ* - 6), SAM *Cₜ* ≤ (NC *Cₜ* - 7), SAM *Cₜ* ≤ (NC *Cₜ* - 8) or SAM *Cₜ* ≤ (NC *Cₜ* - 9), even more preferably when SAM *Cₜ* ≤ (NC *Cₜ* - 10), e.g., SAM *Cₜ* ≤ (NC *Cₜ* - 11), SAM *Cₜ* ≤ (NC *Cₜ* - 12), SAM *Cₜ* ≤ (NC *Cₜ* - 13), SAM *Cₜ* ≤ (NC *Cₜ* - 13) or SAM *Cₜ* ≤ (NC *Cₜ-*14), yet more preferably when SAM *Cₜ* ≤ (NC *Cₜ* - 15), e.g., SAM *Cₜ* ≤ (NC *Cₜ* - 16), SAM *Cₜ* ≤ (NC *Cₜ* - 17), SAM *Cₜ* ≤ (NC *Cₜ* - 18) or SAM *Cₜ* ≤ (NC *Cₜ* - 19), still more preferably when SAM *Cₜ* ≤ (NC *Cₜ* - 20), e.g., SAM *Cₜ* ≤ (NC *Cₜ* - 25), SAM *Cₜ* ≤ (NC *Cₜ* - 30) or SAM *Cₜ* ≤ (NC *Cₜ-*35).

Hence, in real-time amplification, when SAM *Cₜ* is not lower (e.g., is same, about same or greater) than NC *Cₜ,* or preferably is not lower by the preferred differentials recited above, the "absence" of the said nucleic acid in the sample can be generally concluded.

As noted above, in real-time amplification systems which do not employ sequence specific probes (e.g., when amplification product is detected using sequence non-specific DNA dyes such as, e.g., SYBR Green I), the specificity of the amplification product can be verified by performing a melting curve analysis and determining the Tm. Presence of specific amplification product can be concluded when the observed Tm is identical or substantially identical (preferably within an interval of ± 3°C, more preferably ± 2°C, yet more preferably ± 1°C, even more preferably ± 0.5°C, and still more preferably ± 0.2°C or ± 0.1 °C) to the calculated and/or experimentally determined melting temperature of the expected amplicon.

Moreover, specific amplification can be preferably concluded when the melting curve analysis of the amplification product displays a single Tm. Alternatively or in addition, specific amplification can be preferably concluded when the amplification product displays a single, expected size upon gel electrophoresis. However, the appearance of non-specific amplification product(s) might to some extent be tolerable in practice, preferably if such non-specific amplification product(s) would constitute less than 50%, more preferably less than 40%, even more preferably less than 30%, still more preferably less than 20%, yet more preferably less than 10%, further more preferably less than 5%, even more preferably less than 2% and most preferably less than 1 %, e.g., less than 0.5% or less than 0.1 %, of the total quantity (w/w) of the amplification product. Such non-specific amplification product(s) may be observed, e.g., by appearance of one or more additional Tm peaks in the melting curve analysis and/or appearance of one or more additional product sizes upon gel electrophoresis.

In a further development of the invention, it is noted that amplicons amplified using the same primer set on nucleic acids originating from different evens may show distinct Tm values, presumably due to some differences in the sequence between such amplicons. By means of example and not limitation, amplicons amplified using primer set SEQ ID NO: 11 and 12 (Table 3) on Bt11 and Bt176 show such a difference in their Tm. Accordingly, the observed Tm value may serve as a further distinguishing information to use in the method of the invention, such that an even more detailed indication of the presence or absence of nucleic acids from such particular events is obtained.

Alternatively or in addition, in a further variation, a particular nucleic acid may be amplifiable using a particular primer set from one or more events, while the same primer would not amplify the corresponding nucleic acid in one or more other events, presumably due to some sequence differences in the primer binding sites. While such situation would necessitate the use of two or more primer sets to amplify the corresponding nucleic acid from different events (and may in that respect be less advantageous than choosing a primer set that amplifies the nucleic acid from all plant events of interest), it may give the advantage of providing additional information about the presence or absence of nucleic acids from particular events in the sample. For example, the Cry1Ab nucleic acid may be advantageously detected from MON810 using the primer set SEQ ID NO: 30 and 31 and from Bt11 and Bt176 using the primer set SEQ ID NO: 11 and 12 (Table 3).

A skilled person can appreciate that amplification reactions, e.g., PCR, may give rise to a non-specific, "primer-dimer" by-product. Such primer-dimer products can be typically readily distinguished from the specific amplification product(s) by their different, most often smaller, size and/or Tm. Hence, a skilled person would be able to recognise and minimise the effect of such artefacts on the analysis of the amplification reaction.

A further issue is the sensitivity of the detection methods used in the present invention, which may be advantageously expressed as "limit of detection" (LOD) thereof, referring herein to the lowest amount or concentration of an analyte (herein of a given nucleic acid to be detected) in a sample which can be reliably detected, although not necessarily quantified. "Reliably detected" as used herein means that samples containing the LOD amount or concentration of the analyte will give a positive outcome in ≥ 50% of repeated detections, preferably in ≥ 60%, more preferably in ≥ 70%, even more preferably in ≥ 80%, yet more preferably in ≥ 90%, and most preferably in > 95% or even in ≥ 99% of repeated detections.

Preferably, LOD of detection methods suitable for use in the invention, expressed herein as the LOD copy number of a given nucleic acid of interest in a sample subjected to the detection, may be 10 000 or less, e.g., 9 000 or less, 8 000 or less, 7 000 or less or 6 000 or less, more preferably 5 000 or less, e.g., 4 000 or less, 3 000 or less or 2 000 or less, even more preferably 1 000 or less, e.g., 900 or less, 800 or less, 700 or less or 600 or less, yet more preferably 500 or less, e.g., 400 or less, 300 or less or 200 or less, still more preferably 100 or less, e.g., 90 or less, 80 or less, 70 or less or 60 or less, and most preferably 50 or less, with exemplary preferred embodiments including 40 or less, 30 or less, 20 or less or even 10 or less, such as, e.g., 1, 2, 3, 4, 5, 6, 7, 8 or 9.

As used herein, the term "maize" refers to *Zea mays,* preferably *Zea mays* ssp. *mays,* and includes all plant varieties that can be bred with maize, including wild maize species; the terms "rapeseed", "oilseed rape" or "canola" refer interchangeably to *Brassica napus,* and include all plant varieties that can be bred with rapeseed, including wild rapeseed species; the terms "soya", "soy", "soya bean" or "soybean" refer interchangeably to *Glycine max,* and include all plant varieties that can be bred with soybean, including wild soybean species; the term "rice" refers to *Oryza sativa,* including, without limitation, ssp. *indica* and *japonica,* and includes all plant varieties that can be bred with rice, including wild rice species; the term "sugar beet" refers to *Beta vulgaris,* and include all plant varieties that can be bred with sugar beet, including wild sugar beet species; the term "cotton" refers to *Gossypium* species, preferably *Gossypium hirsutum,* and includes all plant varieties that can be bred with cotton, including wild cotton species; the term "potato" refers to *Solanum tuberosum,* and includes all plant varieties that can be bred with potato, including wild potato species.

Transgenic plant events are referred to herein by their designations as commonly employed in the art and familiar to a skilled person. Nevertheless, by means of further assistance, Table 1 below summarises further information sufficient for specific identification of recited events.

**Table 1. Identification of transgenic plant events.**

| **Event** | **Company** | **Host taxon** | **Unique ID (UI)** |
|---|---|---|---|
| Bt176 | Syngenta | *Zea mays* | SYN-EV176-9 |
| Bt11 | Syngenta | *Zea mays* | SYN-BTØ11-1 |
| MON810 | Monsanto | *Zea mays* | MON-ØØ81 QJ-6 |
| MON863 | Monsanto | *Zea mays* | MON-ØØ863-5 |
| TC1507 | Pioneer Hi-Bred / Dow AgroScience | *Zea mays* | DAS-Ø15Ø7-1 |
| NK603 | Monsanto | *Zea mays* | MON-ØØ6Ø3-6 |
| T25 | Bayer CropScience | *Zea mays* | ACS-ZMØØ3-2 |
| GA21 | Monsanto | *Zea mays* | MON-ØØØ21-9 |
| DAS-59122 | Dow AgroSciences / Pioneer Hi-Bred | *Zea mays* | DAS-DAS-59122-7 |
| MIR604 | Syngenta Seeds | *Zea mays* | SYN-IR6Ø4-5 |
| LY038 | Renessen LLC / Monsanto | *Zea mays* | REN-ØØØ38-3 |
| MON88017 | Monsanto | *Zea mays* | MON88Ø17-3 |
| Topas 19/2 | Bayer CropScience | *Brassica napus* | ACS-BNØØ7-1 |
| MS1 | Bayer CropScience | *Brassica napus* | ACS-BNØØ4-7 |
| RF1 | Bayer CropScience | *Brassica napus* | ACS-BNØØ1-4 |
| RF2 | Bayer CropScience | *Brassica napus* | ACS-BNØØ2-5 |
| RF3 | Bayer CropScience | *Brassica napus* | ACS-BNØØ3-6 |
| MS8 | Bayer CropScience | *Brassica napus* | ACS-BNØØ5-8 |
| GT73 | Monsanto | *Brassica napus* | MON-ØØØ73-7 |
| T45 | Bayer CropScience | *Brassica napus* | ACS-BNØØ8-2 |
| Liberator pHoe6/Ac | Bayer CropScience | *Brassica napus* | ACS-BN010-4 |
| GS40/90pHoe6/Ac | Bayer CropScience | *Brassica napus* | ACS-BN010-4 |
| MS1/RF1 | Bayer CropScience | *Brassica napus* | ACS-BNØØ4-7 x ACS-BNØØØ1-4 |
| MS1/RF2 | Bayer CropScience | *Brassica napus* | ACS-BNØØ4-7 x ACS-BNØØ2-5 |
| MS8/RF3 | Bayer CropScience | *Brassica napus* | ACS-BNØØ5-8 x ACS-BNØØ3-6 |
| OXY235 | Bayer CropScience | *Brassica napus* | ACS-BNØ11-5 |
| MON 40-3-2 | Monsanto | *Glycine max* | MON-Ø4Ø32-6 |
| MON89788 | Monsanto | *Glycine max* | MON-89788-1 |
| A2704-12 | Bayer CropScience | *Glycine max* | ACS-GMQØØ5-3 |
| A5547-127 | Bayer CropScience | *Glycine max* | ACS-GMQØØ6-4 |
| LL62 | Bayer CropScience | *Oryza sativa* | ACS-OSØØ2-5 |
| LL06 | Bayer CropScience | *Oryza sativa* | ACS-OSØØ1-4 |
| T120-7 | Bayer CropScience | *Beta vulgaris* | ACS-BVØØ1-3 |
| H7-1 | KWS SAAT AG / Monsanto | *Beta vulgaris* | KM-ØØH71-4 |
| A5-15 | DANISCO Seed; DLF Trifolium A/S; Monsanto | *Beta vulgaris* | DLF-0A515-7 |
| LL cotton 25 | Bayer CropScience | *Gossypium hirsutum* | ACS-GHØØ1-3 |
| MON 1445 | Monsanto | *Gossypium hirsutum* | MON-ØØ1445-2 |
| MON 531 | Monsanto | *Gossypium hirsutum* | MON-ØØ531-6 |
| MON 15985 | Monsanto | *Gossypium hirsutum* | MON-15985-7 |
| EH-92-527-1 | Amylogen HB (BASF Plant Science | *Solanum tuberosum* | BPS-25271-9 |

The above listed unique identifiers (UI) represent a system for unique classification of transgenic plant events set in place by the Organisation for Economic Co-operation and Development (OECD) and described in its publications ENV/JM/MONO(2002)7: "OECD Guidance for the Designation of a Unique Identifier for Transgenic Plants", of October 20, 2004, and ENV/JM/MONO(2002)7/REV1 of November 7, 2006. The said unique identifiers are used, recognised and assigned internationally, including in Europe (see, e.g., Commission Regulation (EC) No 65/2004 of January 14, 2004 establishing a system for the development and assignment of unique identifiers for genetically modified organisms).

Detailed information regarding the above and further transgenic plant events of interest, such as, e.g., information on host taxons, transforming constructs, inserted sequences, etc. can be accessed through publicly available portals of various regulatory authorities including, e.g., the OECD BioTrack Product Database (http://www2.oecd.org/biotech/default.aspx), the US FDA (http://www.cfsan.fda.gov/), the European Community Register of GM Food and Feed (http://ec.europa.eu/food/dyna/gm_register/index_en.cfm), the Agbios database (www.agbios.com), or the EC GMO Compass database (http://www.gmo-compass.org/), as well as in scientific literature, etc. A skilled person is well-aware and can use such database sources.

In addition, event-specific detection methods and construct-specific detection methods (e.g., see *Codex Alimentarium)* are available and allow to unambiguously distinguish the above and further events. For example, the European Community Reference Laboratory (CRL) (http://gmo-crl.jrc.it/; http://gmo-crl.jrc.it/statusofdoss.htm) lists validated methods for event-specific detection, as provided by manufacturers, described in scientific literature or developed and by the CRL itself.

As noted above, the invention allows to conclude the presence or absence in a sample of material derived from select events, crosses thereof, or related events thereof.

The term "crosses thereof' in this context refers to progeny obtained by one or more subsequent crossings of two or more compatible events (i.e., events capable of being crossed, particularly events belonging to the same taxon), such that the said resulting progeny comprises transgenic inserts from the two or more parental events being crossed.

The term "related events thereof" in this context refers to events that have been generated by introducing into the same taxons the same transforming constructs as that of the respective recited events, but are nevertheless distinct from the recited events, e.g., may typically differ in the number and/or site of genomic integrations of the transforming construct. Often, such events may themselves be subject to authorisation. In other instances, such related events may be present as unwanted contaminants. Hence, by detecting genetic elements from within the transforming constructs, the method of the invention advantageously allows to detect such events.

As noted above, the methods of the invention involve detection of the presence or absence in samples of one or more nucleic acids comprising or consisting of those listed under a) - u) as defined above.

In this respect, the recitation "a nucleic acid derived from and specific for" a given taxon, such as defined for nucleic acids listed under a) "Zm", b) "Bn", c) "Gm", o) "Or", p) "Bv", q) "Gs", and t) "St", means that the said nucleic acid in the sample originates from the genetic material, preferably genomic DNA, of the said given taxon and is not present - and thus would not be detectable using the chosen detection method - in the genetic material originating from other taxons, such as the remaining taxons listed above. By means of example and not limitation, said nucleic acid derived from the given taxon may either have no homologues in other taxons or its homologues in other taxons may have sufficiently distinct sequences such that they would not be detected by the chosen detection method. For example, at a site of detection, e.g., at a site to which of a probe or one or more amplification primers would hybridise within the given nucleic acid, such homologues may show less than 100% sequence identity, preferably less than 95%, even more preferably less than 90%, yet more preferably less than 85%, still more preferably less than 80%, and most preferably less than 75%, in preferred exemplary embodiments less than 70%, less than 65%, less than 60%, less than 55% or less than 50% sequence identity with the given nucleic acid.

Further, the term nucleic acid "derived from" as used especially in a) - u) means that the said nucleic acids originate from their respective taxons, genetic elements, genes, open reading frame, etc., albeit they - as found in the sample - may in part structurally differ therefrom.

For instance, downstream processing of plant material may cause alterations in nucleic acid (e.g., DNA) structure, and perhaps most notably may cause fragmentation thereof. In addition, sometimes functional fragments or variants of full-length elements may be used in transgenic plants. Accordingly, nucleic acids "derived from" those recited under a) - u) above may be, e.g., fragments thereof, insofar such fragments allow for their specific attribution to the original nucleic acids. For example, such fragments may include at least 15 continuous bp of the sequence from which they are derived, preferably at least 20 continuous bp, e.g., at least 30 or at least 40 continuous bp, more preferably at least 50 continuous bp, e.g., at least 60 or at least 70 continuous bp, even more preferably at least 80 continuous bp, e.g., at least 90 continuous bp, yet more preferably at least 100 continuous bp, e.g., at least 150 continuous bp, still more preferably at least 200 continuous bp, e.g., at least 300 continuous bp, at least 400 continuous bp or at least 500 continuous bp or more and may even include full-length elements.

Other nucleic acids modifications expected from downstream processing of plant material, e.g., partial deamination, etc., are also contemplated under the term "derived from".

In a preferred embodiment, the nucleic acid a) "Zm" is derived from the endogenous alcohol dehydrogenase I gene (*adh-1*) of *Zea mays.* An exemplary but non-limiting sequence of the *adh-1* gene of *Zea mays* is found under accession number AF123535.1 in the GenBank database (http://www.ncbi.nlm.nih.gov/).

In a further preferred embodiment, the nucleic acid b) "Bn" is derived from the endogenous acetyl-CoA carboxylase gene (*acc*) or the endogenous cruciferin gene of *Brassica napus.* An exemplary but non-limiting sequence of the *acc* gene of *Brassica napus* is found under accession number X77576.1 in the GenBank database; an exemplary sequence of the cruciferin gene of *Brassica napus* is found under accession number X14555.1 in the GenBank database.

In a further preferred embodiment, the nucleic acid c) "Gm" is derived from the endogenous lectin gene *(lec)* of *Glycine max.* An exemplary but non-limiting sequence of the *lec* gene of *Glycine max* is found under accession number K00821.1 in the GenBank database.

In a further preferred embodiment, the nucleic acid o) "Or" is derived from the endogenous phospholipase D gene *(pld)* of *Oryza sativa.* An exemplary but non-limiting sequence of the *pld* gene of *Oryza sativa* is found under accession number AB001919.

In a further preferred embodiment, the generic plant-derived nucleic acid is derived from the endogenous chloroplastic RBCL gene *(rbcl)* of *Zea mays.* An exemplary but non-limiting sequence of the *rbcl* gene of *Zea mays* is found under accession number Z11973.1 in the GenBank database.

A skilled person would be capable of designing and verifying specific probes or primer pairs from the above cited sequences for use in the detection steps of the present method.

The references to sequence elements and genes recited in this specification for the nucleic acids listed under d) - n), r) and u) are known to a skilled person in the field of genetic modification of plants. Hence, a skilled person understands to what sequences these designations refer and would as well appreciate the level of alterations to these sequences that is common in generation of transgenic plants.

In an embodiment, the nucleic acid present in a transgenic event may include the open reading frame or a functional part thereof (i.e., a part achieving the desired effect in the transgenic plant) of the genes recited herein, esp. the genes listed under f) - n), r) and u) above.

Nevertheless, by means of further clarification and not limitation, we here under list exemplary sequences for the particular genes and elements referred to under d) - n), r) and u), and an exemplary generic plant-derived nucleic acid:

| Sequence element / gene | GenBank accession number |
|---|---|
| CMV p35S promoter | V00140 (entire CAMV genome) |
| NOS terminator | V00087.1 |
| Cry1Ab ORF | AF465640 |
| bar gene ORF | AY346130.1 |
| *pat* gene ORF | DQ156557.1 |
| *EPSPS* gene ORF | AY125353.1 |
| modified *Cry3A* gene ORF | AR836206.1 |
| Glb1 promoter | CS155614.1 |
| *Cry38b1* gene ORF | CS410008.1 |
| *Bxn* gene ORF | E01313.1 |
| Cry1Ac gene ORF | EF094884.1 |
| Cry2Ab2 gene ORF | DQ361266.1 |
| Gbss gene ORF | X58453.1 |
| *Zea mays* chloroplast rbcL gene | Z11973.1 |

It shall be appreciated that nucleic acids actually present in transgenic plant events can comprise sequences which may differ to some extent from the above exemplary sequences. For example, such differences may include base deletions, additions and/or substitutions, truncations (e.g., inclusion of functional fragments), fusions to other elements (e.g., fusion to membrane transport or organelle sorting signals), etc.

The actual sequences of the above and other elements found in the transgenic events have been in many instances determined and are accessible via GenBank and other sequence databases. Accordingly, sequence alignments shall be performed in order to identify regions within the above nucleic acids most suitable for derivation of probes or amplicons therefrom.

Hence, a probe or an amplicon (and corresponding primer set) can be advantageously derived from a portions of each of the above nucleic acids which is found in most transgenic events and/or which shows the highest sequence identity between the various transgenic events. Such probe or primer pair will increase the chance of detection of that particular nucleic acid from various transgenic events. A skilled person can follow this instruction to perform the necessary sequence alignments.

As set out in the Summary section, the invention also provides an advantageous manner of concluding the potential presence or absence of material from one or more transgenic plant events of interest to be used in step (2) of the above methods. In particular, the results obtained for the sample in step (1) are represented as a set G_{SAM} symbolising a collection of those nucleic acids, chosen from those comprising or consisting of the ones listed under a) - u) above, that were detected as present in the sample; and comparing the said set G*_{SAM}* with one or more sets of interest G*ₓ* representing the individual events to be evaluated. The sets of interest Gₓ consist of, i.e., represent a collection of, those nucleic acids chosen from ones comprising or consisting of those listed under a) - u) above that are found in the respective events and thus would be detected in step (1) if the sample contained material derived from the respective events, or a cross involving such, or a related event.

The following Table 2 lists which nucleic acids of those listed under a) - u) above are present in particular events of interest:

**Table 2. Features of events.**

| **Event** | **a** | **b** | **c** | **d** | **e** | **f** | **g** | **h** | **i** | **j** | **k** | **l** | **m** | **n** | **O** | **p** | **q** | **r** | **s** | **t** | **u** |
|---|---|---|---|---|---|---|---|---|---|---|---|---|---|---|---|---|---|---|---|---|---|
| Bt176 | + | - | - | + | - | + | + | - | - | - | - | - | - | - | - | - | - | - | - | - | - |
| Bt11 | + | - | - | + | + | + | - | + | - | - | - | - | - | - | - | - | - | - | - | - | - |
| Bt10 | + | - | - | + | + | + | - | + | - | - | - | - | - | - | - | - | - | - | - | - | - |
| MON810 | + | - | - | + | + | + | - | - | - | - | - | - | - | - | - | - | - | - | - | - | - |
| MON863 | + | - | - | + | + | - | - | - | - | - | - | - | - | - | - | - | - | - | - | - | - |
| TC1507 | + | - | - | + | - | - | - | + | - | - | - | - | - | - | - | - | - | - | - | - | - |
| NK603 | + | - | - | + | + | - | - | - | + | - | - | - | - | - | - | - | - | - | - | - | - |
| T25 | + | - | - | + | - | - | - | + | - | - | - | - | - | - | - | - | - | - | - | - | - |
| GA21 | + | - | - | - | + | - | - | - | - | - | - | - | - | - | - | - | - | - | - | - | - |
| DAS-59122 | + | - | - | + | - | - | + | - | - | - | - | - | - | - | - | - | - | - | - | - | - |
| MIR604 | + | - | - | - | + | - | - | - | - | + | - | - | - | - | - | - | - | - | - | - | - |
| LY038 | + | - | - | + | + | - | - | - | - | - | + | + | - | - | - | - | - | - | - | - | - |
| MON88017 | + | - | - | + | + | - | - | - | + | - | - | - | + | - | - | - | - | - | - | - | - |
| Topas 19/2 | - | + | - | + | - | - | + | - | - | - | - | - | - | - | - | - | - | - | - | - | - |
| MS1 | - | + | - | - | + | - | + | - | - | - | - | - | - | - | - | - | - | - | - | - | - |
| RF1 | - | + | - | - | + | - | + | - | - | - | - | - | - | - | - | - | - | - | - | - | - |
| RF2 | - | + | - | - | + | - | + | - | - | - | - | - | - | - | - | - | - | - | - | - | - |
| RF3 | - | + | - | - | + | - | + | - | - | - | - | - | - | - | - | - | - | - | - | - | - |
| MS8 | - | + | - | - | + | - | + | - | - | - | - | - | - | - | - | - | - | - | - | - | - |
| GT73 | - | + | - | - | - | - | - | - | + | - | - | - | - | - | - | - | - | - | - | - | - |
| T45 | - | + | - | + | - | - | - | + | - | - | - | - | - | - | - | - | - | - | - | - | - |
| Liberator pHoe6/Ac | - | + | - | + | - | - | - | + | - | - | -- | - | - | - | - | - | - | - | - | - | - |
| GS40/90pHoe6/Ac | - | + | - | + | - | - | - | + | - | - | - | - | - | - | - | - | - | - | - | - | - |
| MS1/RF1 | - | + | - | - | + | - | + | - | - | - | - | - | - | - | - | - | - | - | - | - | - |
| MS1/RF2 | - | + | - | - | + | - | + | - | - | - | - | - | - | - | - | - | - | - | - | - | - |
| MS8/RF3 | - | + | - | - | + | - | + | - | - | - | - | - | - | - | - | - | - | - | - | - | - |
| OXY235 | - | + | - | + | | - | - | - | - | - | - | - | - | + | - | - | - | - | - | - | - |
| MON 40-3-2 | - | - | + | + | + | - | - | - | + | - | - | - | - | - | - | - | - | - | - | - | - |
| MON89788 | - | - | + | - | - | - | - | - | + | - | - | - | - | - | - | - | - | - | - | - | - |
| A2704-12 | - | - | + | + | - | - | - | + | - | - | - | - | - | - | - | - | - | - | - | - | - |
| A5547-127 | - | - | + | + | - | - | - | + | - | - | - | - | - | - | - | - | - | - | - | - | - |
| LL62 | - | - | - | + | - | - | + | - | - | - | - | - | - | - | + | - | - | - | - | - | - |
| LL06 | - | - | - | + | - | - | + | - | - | - | - | - | - | - | + | - | - | - | - | - | - |
| LL601 | - | - | - | + | + | - | + | - | - | - | - | - | - | - | + | - | - | - | - | - | - |
| T120-7 | - | - | - | + | - | - | - | + | - | - | - | - | - | - | - | + | - | - | - | - | - |
| H7-1 | - | - | - | + | - | - | - | - | + | - | - | - | - | - | - | + | - | - | - | - | - |
| A5-15 | - | - | - | + | + | - | - | - | + | - | - | - | - | - | - | + | - | - | - | - | |
| LL cotton 25 | - | - | - | + | + | - | + | - | - | - | - | - | - | - | - | - | + | - | - | - | - |
| MON 1445 | - | - | - | + | + | - | - | - | + | - | - | - | - | - | - | - | + | - | - | - | - |
| MON 531 | - | - | - | + | + | - | - | - | - | - | - | - | - | - | - | - | + | + | - | - | - |
| MON 15985 | - | - | - | + | + | - | - | - | - | - | - | - | - | - | - | - | + | + | + | - | - |
| EH-92-527-1 | - | - | - | - | + | - | - | - | - | - | - | - | - | - | - | - | - | - | - | + | + |

As already explained:
- if Gₓ equals *G_{SAM}* (G_{x =} *G_{SAM}),* then material derived from the transgenic plant event X, or from a cross thereof, or from an event related thereto, is potentially present in the sample;
- if Gₓ is a proper subset of G*_{SAM}* (G*ₓ* c G*_{SAM}),* then material derived from transgenic plant event X, or from a cross thereof, or from an event related thereto, is potentially present in the sample;
- if Gₓ does not equal *G_{SAM}* and Gₓ is not a proper subset of *G_{SAM},* (Gₓ ≠ *G_{SAM}* and Gₓ ⊄ *G_{SAM}),* then material derived from transgenic plant event X, or from a cross thereof, or from an event related thereto, is absent from the sample.

In a further preferred embodiment, if Gₓ equals *G_{SAM},* and if no one set or the sum of two or more sets representing events (in particular, sets representing events chosen from a group comprising or consisting of: *G_{Bt176}, G_{Bt11}, G_{Bt10}, G_{MON810}, G_{MON863}, G_{TC15073} G_{NK6033} G_{T253} G_{GA213} G_{DAS-591223} G_{MIR604}, G_{LY038}, G_{MON88017}, G*_{*Topas 19*/}*₂₃ G_{MS1}, G_{RF1}, G_{RF2}, G*_{*MS1*/}*_{RF1}, G*_{*MS1*/}*_{RF2}, G_{MS83} G_{RF33} G*_{*MS8*/}*_{RF33} G_{GT73}, G_{T45}, G*_{*Liberator pHoe6*/}*_{Ac}, G*_{*GS40*/*90pHoe6*/}*_{Ac3} G_{OXY2353} G_{MON40-3-2}, G_{MON89788}, G_{A2704-12}*, *G_{A5547-1273}* GLL62, *G_{LL063} G_{LL6013} G_{T120-7}, G_{H7-1}, G_{A5-153} G_{LL cotton 25}, G_{MON1445}, G_{MON5313} G_{MON15985}* and *G_{EH92-527-1)}* other than Gx equals Gₓ, then material derived from transgenic plant event X or from a cross thereof, or from an event related thereto, is present in the sample.

In a further development of this manner of evaluation, nucleic acids comprising or consisting of those listed under a) - u) above are assigned respective unique values. Hence, the set G*_{SAM}* is then assigned a value "VG*_{SAM}*" being a multiple of the unique values assigned to those nucleic acids detected in step (1) as being present in the sample; and a set of interest G*ₓ* is assigned a value "VG*_{X}*" being a multiple of the unique values assigned to those nucleic acids chosen from ones comprising or consisting of those listed under a) - u) above that are found in the said event and thus would be detected in step (1).

Accordingly, in a preferred example, nucleic acids "Zm", "Bn", "Gm", "p35S", "tNOS", "Cry1Ab", "PAT/bar", "PAT/pat", "CP4-EPSPS", "mCry3A", "cordap A", "Glb1", "Cry3Bb1", "Bxn", "Or", "Bv", "Gs", "Cry1Ac", "Cry2ab2", "St" and "Gbss" would be assigned respective unique values "V_{zm}", *"*V*_{Bn}",* "V_{Gm}", *"*Vp*_{35S}",* "V_{tNOS}", *"*V*_{Cry1Ab}", "*V_{*PAT*/}*_{bar}", "*V_{*PAT*/}*ₚₐₜ", "*V*_{CP4-EPSPS}",* "V_{mCty3A}", *"*V*_{cordap A}", "*V*_{Glb1}", "*V*_{Cry3Bb1}", "*V*_{Bxn}", "*V*_{Or}",* "V_{Bv}", "V_{Gs}", *"*V*_{CrylAc}", "*V*_{Cry2ab2}*", *"*V*ₛₜ"* and "V_{Gbss}",
and each set of interest Gₓ would be assigned a respective value "VGₓ' chosen from values of the group comprising or consisting of values "VG_{Bt176}", "VG_{Bt11}", "VG_{Bt10}", "VG_{MON810}", "VG_{MON863}" "VG_{TC1507}", "VG_{NK603}"_{,} "V"G_{T25}"_{,} "VG_{GA21}", "VG_{DAS-59122}", *"*VG*_{MIR604}",* "VG_{LY038}", *"*VG*_{MON88017}"_{,}* "VG_{*Topas* 19/2}", "VG_{MS1}", "VG_{RF1}", "VG_{RF2}", "VG_{MSMRF1}", *"*VG_{*MS1*/}*_{RF2}"_{,}* "VG_{MS8}", "VG_{RF3}"_{,} *"*VG_{*MS8*/}*_{RF3}",* "VG_{GT73}", "VG_{T45}", *"*VG_{*Liberator pHoe6*/*Ac*}", *"*VG_{*GS40*/}*_{90pHoe6lAc}", "*VG*_{OXY235}"*, *"*VG*_{MON40-3-2}", "*VG*_{MON89788}", "*VG*_{A2704-12}", "*VG*_{A5547-127}", "*VG*_{LL62}", "*VG*_{LL06}", "*VG*_{LL601}",* "VG_{*T120*-*7*}", "VG*_{H7-1}*", "VG*_{A5-15}*", *"*VG*_{LL cotton 25}*", *"*VG*_{MON1445}",* "VG*_{MOM531}*", "VG*_{MON15985}*" Or *"*VG*_{EH92-527-1}"*, wherein:
- VG*_{Bt176}* = V_{Zm} × V*_{p35S}* × V*_{Cry1Ab}* × V_{*PAT*/}*_{bar},*
- VG_{Bt11} = V*_{Zm}* × V*_{p35S}* × V_{tNOS} × V*_{Cry1Ab}* × V_{*PAT*/}*ₚₐₜ,*
- VG*_{Bt10}* = V*_{Zm}* × V*_{p35S}* × V_{tNOS} × V*_{Cry1Ab}* × V_{*PAT*/}*ₚₐₜ,*
- VG*_{MON810}* = V_{zm} × V*_{p35S}* × V_{tNOS} × V*_{Cry1Ab},*
- VG_{MON863} *=* V*_{Zm}* × V*_{p35S}* × V*_{tNOS},*
- VG*_{TC1507} =* V*_{Zm}* × V*_{p35S}* × V_{*PAT*/}*ₚₐₜ,*
- VG*_{NK603}* = V*_{Zm}* × V*_{p35S}* × V_{tNOS} × V*_{CP4-EPSPS},*
- VG_{T25} *=* V*_{Zm}* × V*_{p35S}* × V_{*PAT*/}*ₚₐₜ,*
- VG*_{GA21}* = V*_{Zm}* × *V_{tNOS},*
- VG*_{DAS-59122}* = V*_{Zm}* × V*_{p35S}* × *V*_{*PAT*/*bar3*}
- VG*_{MIR604}* = V*_{Zm}* × V*_{tNOS}* × V*_{mCry3A},*
- VG*_{LY038}* = V*_{Zm}* × V*_{p35S}* × V_{tNOS} × *_{VcordapA}* × V*_{Glb1},*
- VG*_{MON88017}* = V*_{Zm}* × V_{p35S} × V_{tNOS} × V*_{CP4-EPSP}* × V*_{Cry3Bb13} ,*
- VG_{*Topas* 19/2} = V*_{Bn}* × V*_{p35S}* × V_{*PAT*/}*ₚₐₜ,*
- VG*_{MS1}* = V*_{Bn}* × V*_{tNOS}* × V_{*PAT*/}*_{bar},*
- VG*_{RF1}* = V*_{Bn}* × V*_{tNOS}* × V_{*PAT*/*bar,*}
- VG*_{RF2}* = V*_{Bn}* × V*_{tNOS}* × V_{*PAT*/}*_{bar},*
- VG_{MS8} = V*_{Bn}* × V*_{tNOS}* × V_{*PAT*/}*_{bar},*
- VG*_{RF3}* = V*_{Bn}* × V*_{tNOS}* × V_{*PAT*/}*_{bar},*
- VG_{*MS1*/*RF1*} = V*_{Bn}* × V_{tNOS} × V_{*PAT*/}*_{bar},*
- VG_{*MS1*/*RF2*} = V*_{Bn}* × V*_{tNOS}* × V_{*PAT*/}*_{bar},*
- VG_{*GMSB*/*RF3*} = V*_{Bn}* × V*_{tNOS}* × V_{*PAT*/}*_{bar},*
- VG*_{GT73}* = V*_{Bn}* × V*_{CP4-EPSPS},*
- V_{G*T45*} = V*_{Bn}* × V*_{p35S}* × V_{*PAT*/}*ₚₐₜ,*
- VG_{*Liberator pHoes*/*Ac*} = V*_{Bn}* × V*_{p35S}* × V_{*PAT*/}*ₚₐₜ,*
- VG_{*GS40*/*90pHoe6*/*Ac*} = V*_{Bn}* × V*_{p35S}* × V_{*PAT*/}*ₚₐₜ,*
- VG*_{OXY235}* = V*_{Bn}* × V*_{p35S}* × V*_{Bxn},*
- VG*_{MON40-3-2}* = V*_{Gm}* × V*_{p35S}* × V_{tNOS} × V*_{CP4-EPSPS},*
- VG_{MON89788} = V*_{Gm}* × V*_{CP4-EPSPS},*
- VG*_{A2704-12}* = V*_{Gm}* × V*_{p35S}* × V_{*PAT*/*pat,*}
- VG*_{A5547-127}* = V*_{Gm}* × V*_{p35S}* × V_{*PAT*/*pat*},
- VG*_{LL62}* = V*_{Or}* × V*_{p35S}* × V_{*PAT*/}*_{bar},*
- VG*_{LL06}* = V*_{Or}* × V*_{p35S}* × V_{*PAT*/*bar*},
- VG*_{LL601}* = V*_{Or}* × V*_{p35S}* × V*_{tNOS}* × V_{*PAT*/}*_{bar},*
- VG*_{T120-7}* = V*_{Bv}* × V*_{p35S}* × V_{*pAT*/}*ₚₐₜ,*
- VG*_{H7-1}* = V*_{Bv}* × V*_{p35S}* × V*_{CP4-EPSPS}*,
- VG*_{A5-15}* = V*_{Bv}* × V*_{p35S}* × V*_{tNOS}* × V*_{CP4-EPSPS},*
- VG_{*LL cotton* 25} = V*_{Gs}* × V*_{p35S}* × V*_{tNOS}* × V_{*PAT*/}*_{bar},*
- VG*_{MON1445}* = V*_{Gs}* × V*_{p35S}* × V*_{tNOS}* × V*_{CP4-EPSPS},*
- VG*_{MON531}* = V*_{Gs}* × V*_{p35S}* × V*_{tNOS}* × V*_{Cry1Ac}*,
- VG*_{MON15985}* = V*_{Gs}* × V*_{p35S}* × V*_{tNOS}* × V*_{Cry1Ac}* × V*_{Cry1Ac}*,
- or VG*_{EH92-527-1}* = V*_{St}* × V*_{tNOS}* × V*_{Gbss}.*

Consequently, step (iii) of this embodiment would comprise performing for each set of interest Gₓ logical operations:
- if VG_{SAM} / VG*ₓ* equals 1, then material derived from transgenic plant event *X* or from a cross thereof, or from an event related thereto, is potentially present in the sample;
- if VG*_{SAM}* / VG*ₓ* equals one value or a multiple of two or more values chosen from a group comprising or consisting of values V*_{Zm},* V_{Bn}, V*_{Gm},* V*_{p35S},* V*_{tNOS},* V_{Cry1Ab}, V_{*PAT*/}*_{bar},* V_{*PAT*/}*ₚₐₜ, V_{CP4-EPSPS},* V*_{mCry3A},* V*_{cordap A},* V *_{Glb1},* V*_{Cry3Bb1}, V_{Bxn},* V*_{Or},* V*_{Bv},* V*_{Gs},* V*_{Cry1Ac},* V *_{C}ry_{2ab2},* V*_{St}* and V*_{Gbss}*, then material derived from transgenic plant event *X* or from a cross thereof, or from an event related thereto, is potentially present in the sample;
- if VG*_{SAM}* / VG*ₓ* does not equal 1 and does not equal one value or a multiple of two or more values chosen from a group comprising or consisting of values V*_{Zm}*, V*_{Bn}*, V*_{Gm},* V*_{p35S},* V*_{tNOS}*, V*_{Cry1Ab}*, V_{*pAT*/}*_{bar},* V_{*pAT*/}*ₚₐₜ,* V*_{CP4-EPSPS},* V_{*mC*r}*_{y3A}, V_{cordap A},* V*_{Glb1},* V*_{Cry3Bb1},* V*_{Bxn},* V*_{Or},* V*_{Bv},* V*_{Gs}*, V*_{Cry1Ac},* V*_{Cry2ab2},* V*_{St}* and V*_{Gbss},* then material derived from transgenic plant event *X* or from a cross thereof, or from an event related thereto, is absent from the sample.

In a further preferred variant of the above embodiment, if VG_{SAM} / VGₓ equals 1, and if no one value or the multiple of two or more values of sets representing events (in particular, values chosen from a group comprising or consisting of: VG*_{Bt176}*, VG*_{Bt11},* VG*_{Bt10}*, VG*_{MON810}*, VG*_{MON863}*, VG*_{TC1507}*, VG*_{NK603},* VG*_{T25}*, VG*_{GA21},* VG*_{DAS-59122},* VG*_{MlR604}*, VG*_{LY038},* VG*_{MON88017},* VG*_{Topas} 19*/*2*, VG*_{MS1}*, VG*_{RF1}*, VG*_{RF2}*, VG_{*MS1*/}*_{RF1},* VG_{*MS1*/}*_{RF2},* VG*_{MS8},* VG*_{RF3},* VG_{*MSB*/}*_{RF3},* VG*_{GT73},* VG*_{T45}*, VG_{*Liberator pHoe6*/}*_{Ac},* VG_{*GS40*/*90pHoe6*/}*_{Ac},* VG*_{OXY235},* VG*_{MON40-3-2},* VG*_{MON89788},* VG*_{A2704-12},* VG*_{A5547-127},* VG*_{LL62}*, VG*_{LL06}*, VG*_{LL601},* VG*_{T120-7}*, VG_{H7-1}, VG*_{A5-15}*, VG*_{LL cotton 25}*, VG*_{MON1445},* VG*_{MON531},* VG*_{MON15985}* and VG*_{EH92-527-1}*) other than VG*ₓ* equals VG*ₓ*, then material derived from transgenic plant event *X* or from a cross thereof, or from an event related thereto, is present in the sample.

In a further preferred embodiment, the unique values assigned to the nucleic acids comprising or consisting of those listed under a) - u), i.e., values chosen from the group comprising or consisting of V*_{Zm},* V*_{Bn},* V*_{Gm}*, V*_{p35S}*, V*_{tNOS},* V*_{Cry1Ab},* V_{*PAT*/}*_{bar},* V*_{PATlpat},* V*_{CP4-EPSPS3}*, V*_{mCry3A}, V_{cordap A},* V*_{Glb1}*, V_{C*ry3Bb1*}, V*_{Bxn},* V*_{Or},* V*_{BV},* V*_{Gs}*, V*_{Cry1Ac},* V*_{Cry2ab2},* V*_{St}* and V*_{Gbss},* are unique prime numbers, and the step (iii) comprises performing for each set of interest G*ₓ* logical operations,
- if VG*_{SAM}* / VGₓ equals 1, then material derived from transgenic plant event *X* or from a cross thereof, or from an event related thereto, is potentially present in the sample;
- if VG*_{SAM}* / VG*ₓ* is an integer greater than 1, then material derived from transgenic plant event *X* or from a cross thereof, or from an event related thereto, is potentially present in the sample;
- if VG*_{SAM}* / VG*ₓ* is not an integer, then material derived from transgenic plant event *X* or from a cross thereof, or from an event related thereto, is absent from the sample.

In a preferred embodiment, the above calculation steps of the method step (2) are carried out by a computing device, e.g., a calculator or a computer; and in further aspects the invention also contemplates a computing device performing the above calculations, a data carrier (e.g., a diskette, CD-ROM, etc.) comprising instructions for a programmable computing device to carry out the step (2) of the method of the invention, including the above calculations, a kit comprising such data carrier alongside one or more reagents also useful in the methods of the invention.

As mentioned, the method of the invention may preferably evaluate the presence or absence of nucleic acids of interest, especially nucleic acids chosen from the group comprising or consisting of those listed under a) - u) above, using amplification of amplicons therefrom.

It can be appreciated that a skilled person is in general capable of designing primer sets for specific amplification from known nucleic acid sequences. Nevertheless, the inventors in Table 3 also provide advantageous primer sets to amplify the nucleic acids of the invention, and methods using such primers. These primer sets can advantageously achieve amplification from the respective nucleic acids as present in the relevant plant events and from variously processed plant material.

**Table 3. Primer sequences.**

| **Nucleic acid** | **Primers** |
|---|---|
| "Zm" | Fwd: 5' CgTCgTTTCCCATCTCTTCCTCC 3' (SEQ ID NO: 1) |
| (adh-1) | Rev: 5' CCACTCCgAgACCCTCAgTC 3' (SEQ ID NO: 2) |
| "Bn" | Fwd: 5' GGCGCAGCATCGGCT 3' (SEQ ID NO: 3) |
| (ACC) | Rev: 5' GAGAATGAGGAGGACCAAGCTC 3' (SEQ ID NO: 4) |
| "Gm" | Fwd: 5' CATTACCTATgATgCCTCCACC 3' (SEQ ID NO: 5) |
| (lectin) | Rev: 5' AAgCACgTCATgCgATTC 3' (SEQ ID NO: 6) |
| | Rev': 5' AAgCACgTCATgCgATTCC 3' (SEQ ID NO: 49) |
| "p35S" | Fwd: 5'-GACAGTGGTCCCAAAGATGG-3' (SEQ ID NO: 7) |
| | Rev: 5'-GTCTTGCGAAGGATAGTGGG-3' (SEQ ID NO: 8) |
| "tNOS" | Fwd: 5'-GATTAGAGTCCCGCAATTATACATTTAA-3' (SEQ ID NO: 9) |
| ("TNOS-D") | Rev: 5'-TTATCCTAGKTTGCGCGCTATATTT-3' (SEQ ID NO: 10) |
| "Cry1Ab" | Fwd: 5'-ACCGGTTACACTCCCATCGA-3' (SEQ ID NO: 11) |
| | Rev: 5'-CAGCACCTGGCACGAACTC-3' (SEQ ID NO: 12) |
| "PAT/bar" | Fwd: 5'-CGTCAACCACTACATCGAGACAA-3' (SEQ ID NO: 13) |
| | Rev: 5'-GTCCACTCCTGCGGTTCCT-3' (SEQ ID NO: 14) |
| "PAT/pat" | Fwd: 5'-CCGCGGTTTGTGATATCGTT-3' (SEQ ID NO: 15) |
| | Rev: 5'-TCTTGCAACCTCTCTAGATCATCAA-3' (SEQ ID NO: 16) |
| "CP4-EPSPS" | Fwd1: 5'-GGCTCTGAGCTTCGTCCTCCTAAGG-3' (SEQ ID NO: 17) |
| | Fwd1': 5'-GGCTCTGAGCTTCGTCCTCTTAAGG-3' (SEQ ID NO: 50) |
| | Fwd2: 5'-ATCAGTGGCTACAGCCTGCAT-3' (SEQ ID NO: 18) |
| | Rev: 5'-GAATGCGGACGGTTCCGGAAAG-3' (SEQ ID NO: 19) |
| "Or" | Fwd: 5' GCTTAGGGAACAGGGAAGTAAAGTT 3' (SEQ ID NO: 20) |
| | Fwd': 5' GCTTAGGGAACAGGGAAGTAAAGT 3' (SEQ ID NO: 51) |
| | Rev: 5' CTTAGCATAGTCTGTGCCATCCA 3' (SEQ ID NO: 21) |
| "CP4-EPSPS" | Fwd: 5' GCATGCTTCACGGTGCAA 3' (SEQ ID NO: 22) |
| | Rev: 5' GGACCTGTGGGAGATAGACTTGTC 3' (SEQ ID NO: 23) |
| "Bn" | Fwd: 5' CAGCTCAACAGTTTCCAAACGA 3' (SEQ ID NO: 24) |
| (cruciferin) | Rev: 5' CGACCAGCCTCAGCCTTAAG 3' (SEQ ID NO: 25) |
| generic plant | Fwd: 5' AGGTCTAADGGRTAAGCTAC 3' (SEQ ID NO: 26) |
| (Rbcl) | Rev: 5' AGYCTTGATCGTTACAAAGG 3' (SEQ ID NO: 27) |
| "tNOS" | Fwd: 5' CGTTCAAACATTTGGCAATAAAG 3' (SEQ ID NO: 28) |
| (TNOS-L) | Rev: 5' AAATGTATAATTGCGGGACTCTAATC 3' (SEQ ID NO: 29) |
| "Cry1Ab" | Fwd: 5' GACATCATCTGGGGYATCTT 3' (SEQ ID NO: 30) |
| | Rev: 5' GCGCTGTTCATGTCGTTGAA 3' (SEQ ID NO: 31) |
| "Cry1Ab" | Fwd: 5' ACGCCTTCCTGGTGCAAA 3' (SEQ ID NO: 47) |
| | Rev: 5' CCTGGTTCCTGGCGAACTC 3' (SEQ ID NO: 48) |

In addition, the invention also provides variant primers that include one or more sequence variations vis-a-vis the primers listed in Table 3, such as, e.g., one or more deletion, insertion and/or substitution, insofar such primers / primer pairs can still achieve adequate amplification of their respective amplicons. Preferably, such variant primers would show at least 85%, more preferably at least 90%, even more preferably at least 95%, and yet more preferably at least 96%, at least 97%, at least 98% or at least 99% sequence identity to the primers listed in Table 2. Sequence alignments and determination of sequence identity can be done, e.g., using the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250).

In addition, the invention also contemplates nested primers and primer pairs suitable for amplification of amplicons from within those amplified from the respective nucleic acids using the primer pairs listed in Table 3.

In a further aspect, the invention provides amplification products obtainable from the respective nucleic acids using the above primers and primer pairs, and particularly using primer pairs set out in Table 3. The said amplification products may be further processed, such as, e.g., isolated, cloned in suitable vectors or plasmids, transformed into recombinant hosts, e.g., suitable bacterial hosts such as E. *coli,* propagates therewith and isolated therefrom, sequenced, etc.

Advantageously, the said amplification products, preferably cloned and re-isolated, and possibly comprised in suitable plasmids or vectors, can be used as positive controls to verify the working of detection steps of the methods of the invention aimed at the detection of the respective nucleic acid in a sample. Alternatively or in addition, such amplification products (cloned or isolated) may be used as calibrators to determine the quantity of particular templates in a sample, e.g., in real-time PCR.

Accordingly, in a further aspect, the invention provides recombinant E. *coli* as deposited under the Budapest Treaty with the Belgian Coordinated Collections of Microorganisms (BCCM) on January 10, 2007 under LMBP accession numbers LMBP 5452, LMBP 5453, LMBP 5454, LMBP 5455, LMBP 5456, LMBP 5457, LMBP 5458, LMBP 5459 and LMBP 5460, on March 6, 2007 under LMBP accession number LMBP 5451, and on April 19, 2007 under LMBP accession numbers LMBP 5587, LMBP 5588, LMBP 5589 and LMBP 5590.

The said recombinant E. *coli* comprise the amplification products obtained on template events using primer sets as listed in Table 4, cloned in the EcoRl sites of the pUC18 cloning vector MCS:

**Table 4**

| **LMBP acc. #** | **Primer pair** | **Amplified on event** | **Insert sequenced (****Fig. 1****)** |
|---|---|---|---|
| LMBP 5460 | SEQ ID NO: 7 | BT11 | SEQ ID NO: 34 |
| | SEQ ID NO: 8 | | |
| LMBP 5456 | SEQ ID NO: 9 | 40-3-2 | SEQ ID NO: 35 |
| | SEQ ID NO: 10 | | |
| LMBP 5452 | SEQ ID NO: 47 | MON810 | SEQ ID NO: 36 |
| | SEQ ID NO: 48 | | |
| LMBP 5453 | SEQ ID NO: 11 | Bt176 | SEQ ID NO: 37 |
| | SEQ ID NO: 12 | | |
| LMBP 5454 | SEQ ID NO: 11 | Bt11 | SEQ ID NO: 38 |
| | SEQ ID NO: 12 | | |
| LMBP 5457 | SEQ ID NO: 13 | BT176 | SEQ ID NO: 39 |
| | SEQ ID NO: 14 | | |
| LMBP 5455 | SEQ ID NO: 15 | BT11 | SEQ ID NO: 40 |
| | SEQ ID NO: 16 | | |
| LMBP 5458 | SEQ ID NO: 26 | Maize Bt11 | SEQ ID NO:41 |
| | SEQ ID NO: 27 | | |
| LMBP 5459 | SEQ ID NO: 26 | OSR (oil seed rape) wt | SEQ ID NO:42 |
| | SEQ ID NO: 27 | | |
| LMBP 5451 | SEQ ID NO: 28 | Bt11 | SEQ ID NO:43 |
| | SEQ ID NO: 29 | | |
| LMBP 5587 | SEQ ID NO: 22 | RRS soybean | SEQ ID NO: 53 |
| | SEQ ID NO: 23 | | |
| LMBP 5588 | SEQ ID NO: 22 | GT 73 | SEQ ID NO: 54 |
| | SEQ ID NO: 23 | | |
| LMBP 5589 | SEQ ID NO: 24 | GT 73 | SEQ ID NO: 55 |
| | SEQ ID NO: 25 | | |
| LMBP 5590 | SEQ ID NO: 51 | WT rice | SEQ ID NO: 52 |
| | SEQ ID NO: 21 | | |

In addition to deposited plasmids and E. *coli* as listed in Table 4, the invention also provides further exemplary plasmids, such as, e.g., pUC18, containing inserts amplified using, e.g.:
- the primer pair SEQ ID NO: 3 and 4 for the *Brassica* acc gene (exemplary insert sequence SEQ ID NO: 44 in Fig. 1 amplified on OSR wt);
- CP4-EPSP primer pairs SEQ ID NO: 18 and 19, and SEQ ID NO: 50 and 19 (exemplary inserts amplified on, respectively, event 40-3-2 and event NK603, to obtain the respective exemplary insert sequences shown as SEQ ID NO: 45 and 46 in Fig. 1) (primer SEQ ID NO: 17 may also be used in conjunction with primer SEQ ID NO: 19; however, primer SEQ ID NO: 17 contains a sequence mismatch at nucleotide 20 compared to SEQ ID NO: 50 the latter being therefore more preferred);
- the primer pair SEQ ID NO: 1 and 2 for the maize adh-1 gene (exemplary insert sequence SEQ ID NO: 32 in Fig. 1 amplified on wild-type maize);
- the primer pairs SEQ ID NO: 5 and 49 for the soybean lectin gene (exemplary insert sequence SEQ ID NO: 33 in Fig. 1 amplified on wild-type soybean).

The invention further provides isolated recombinant plasmids obtainable from the recombinant E. *coli* bacteria listed in Table 4, as well as isolated inserts, preferably EcoRI inserts, thereof. The invention also provides any further recombinant microorganism transformed with the so-isolated plasmids.

A skilled person can also appreciate that the invention may too provide combinations of the said plasmids or inserts thereof, wherein the said combinations are representative of those nucleic acids that one intends to detect in a sample.

In addition, the invention also provides the use of recombinant plasmids or inserts as found in and obtainable from the bacteria of Table 4 for use as positive controls and/or calibrators in the methods of the present invention.

In a further aspects, the invention provides kits of parts for performing the methods of the present invention, i.e., for examining a sample for the potential presence or absence of material derived from one or more transgenic plant events.

In an embodiment, a kit according to the invention can comprise probes suitable for detection of one or more or all nucleic acids listed under a) - u) above.

In an embodiment, a kit according to the invention can comprise primers, preferably primer pairs, suitable for amplification of amplicons from within one or more or all nucleic acids listed under a) - u) as defined above.

In another embodiment, the kit may comprise primers, preferably primer pairs, suitable for amplification of one, more then one, and preferably all of nucleic acids listed under a) - i) above.

In another embodiment, the kit may comprise primers, preferably primer pairs, suitable for amplification of one, more then one, or all of nucleic acids listed under a) - c) above and one, more than one or preferably all of nucleic acids listed under d) - i) above.

In a preferred embodiment, the kit may comprise one or more, or all primers, preferably primer pairs, as defined in Table 3.

In a further embodiment, the kit may also comprise amplification products, e.g., cloned and re-isolated and possibly comprised in suitable plasmids or vectors, that can be used as positive controls and/or calibrators in the methods of the invention, as explained above. For example, precisely measured quantities or dilutions of such amplification products, e.g., cloned and re-isolated and possibly comprised in suitable plasmids or vectors, may be provided for such purposes. As explained above, various combinations of such reagents may also be envisaged.

In a particular embodiment, the kit may comprise one, more than one or all of the E. *coli* organisms as listed in Table 4, and/or plasmids obtainable therefrom, and/or isolated inserts, preferably *EcoRl* inserts, of the said plasmids, or combinations hereof.

In a further embodiment, as already explained, the kit may also comprise a data carrier containing instructions for a programmable computing device to perform the actions of step (2) of the methods of the invention.

Understandably, such kits may comprise further components, such as components useful hybridisation, PCR, detection, etc. A skilled person will appreciate the potential use of such components.

### EXAMPLES

### Example 1: Amplification conditions for select primer pairs listed in Table 2

SYBR Green detection real-time PCR was optimised for amplification of amplicons from nucleic acids "p35S", "tNOS", "Cry1Ab", "PAT/Bar", "PAT/pat" and "CP4-EPSPS" using respective primer pairs listed in Table 2.

DNA was isolated from events containing (positive control) or lacking (negative control) these nucleic acids, including events Bt11, Bt176, MON810, MON40-3-2, TC1507, NK603, MS8/RF3 (see Table 4), using CTAB isolation from leaf tissue material.

PCR reactions contained SYBR Green PCR Master Mix (Diagenode, ref: GMO-GS2X-A300) 1x, Forward primer 250 nM, Reverse primer 250 nM, Template DNA 50 ng in total volume of 20 µl. PCR cycling involved Step 1: 1× [50°C, 120 sec]; Step 2: 1× [95°C 600 sec]; and Step 3: 40x [95°C 15 sec, 60°C 60 sec] with fluorescence acquisition. Applied Biosystems Prism 7700 was used. Melting curve analysis was done at gradient from 50°C to 95°C over 1200 sec.

For all primer pairs, specific amplification products were obtained as shown by a singe specific Tm and single band on agarose gel electrophoresis. The assays had low LOD:
p35S: Tm = 80,5°C; size = 147 bp; LOD = ± 0.016 ng target DNA (± 6 haploid genomes)
tNOS: Tm = 72°C; size = 172 bp; LOD = ± 0.03 ng target DNA (± 25 haploid genomes)
Cry1Ab: Tm = 78.5°C; size = 73 bp; LOD =± 0.06 ng (±12 haploid genomes)
PAT/Bar: Tm = 80°C; size = 69bp; LOD = ± 0.125ng (±50 haploid genomes)
PAT/pat: Tm = 77°C; size = 109 bp; LOD = ± 0.05ng (±20 haploid genomes)
CP4-EPSP: Tm = 85°C; sizes = 94 bp or 124 bp; LOD = ±0.03 ng (25 haploid genomes)

### Example 2: Exemplary simplified method according to the invention

A hypothetical sample was made by adding of Bt176 DNA to unrelated carrier DNA. The sample was screened for the presence or absence of "p35S", "tNOS", "Cry1Ab", "PAT/Bar", "PAT/pat" and "CP4-EPSPS" using the real-time PCR methods of Example 1.

The present simplified method intends to conclude on the potential presence or absence in the sample of material derived from Bt176, Bt11 and NK603.

After testing, the sample is positive for p35S, Cry1Ab and Pat/Bar but not for the other tested nucleic acids. Hence, the sample can be represented as set G_{SAM} ∈ {p35S; Cry1Ab; Pat/bar}. In this exemplary assay (where the presence of *Zea mays* nucleic acids is not examined), the event Bt176 is represented by set G_{Bt176} ∈ {p35S; Cry1Ab; Pat/bar}, event Bt11 by set G_{Bt11} ∈ {p35S; tNOS; Cry1Ab; Pat/pat} and event NK603 by set G_{NK603} ∈ {p35S; tNOS; CP4-EPSP}.

Accordingly, since G_{Bt176} = G_{SAM}, material from event Bt176 may be present in the sample. On the other hand, because G_{Bt11}≠ G_{SAM} and G_{Bt11}⊄ G_{SAM}, material from event Bt11 is not present in the sample. Similarly, because G_{NK603}≠ G_{SAM} and G_{NK603} ⊄ G_{SAM}, material from event NK603 is not present in the sample.

In an alternative representation, "p35S", "tNOS", "Cry1Ab", "PAT/Bar", "PAT/pat" and "CP4-EPSPS" are assigned respective prime values V*_{p35S}* = 3, V*_{tNOS}* = 5, V*_{Cry1Ab} = 7,* V_{*PAT*/}*_{Bar} =* 11, V_{*PAT*/}*ₚₐₜ =* 13 and V*_{CP4-EPSPS} =* 17.

Consequently, the set G_{SAM} is assigned a value VG_{SAM} being a multiple of the respective values assigned to nucleic acids detected therein, i.e., VG_{SAM} = V*_{p35S}* × V*_{Cry1Ab}* × V_{*PAT*/}*_{Bar} =* 231. Moreover, the set G_{Bt176} is assigned a value VG_{Bt176} being a multiple of the values assigned to those of the tested nucleic acids that are found in the event Bt176, i.e., VG_{Bt176} = V*_{p35S}* × V*_{Cry1Ab}* × *V*_{*PAT*/}*_{Bar}=* 231. Analogously, the set G_{Bt11} is assigned a value VG_{Bt11} = V*_{p35S}* ×X V*_{tNOS}* × V*_{Cry1Ab}* × V_{*pAT*/*pat*} = 1365; and the set G_{NK603} is assigned a value VG_{NK603} = Vp_{35S} × V*_{tNOS}* × V*_{CP4-EPSP}* = 255.

Accordingly, because VG_{SAM} / VG_{Bt176} = 1, material from event Bt176 may be present in the sample. On the other hand, because VG_{SAM} / VG_{Bt11} = 0.169, i.e., is not 1 and not an integer greater than 1, material from event Bt11 is not present in the sample. Similarly, because VG_{SAM} / VG_{NK603} = 0.906, i.e., is not 1 and not an integer greater than 1, material from event NK603 is not present in the sample.

## Claims

1. A method to examine a sample for the presence or absence of material derived from one or more transgenic plant events comprising the steps of:
(1) detecting the presence or absence in the sample of nucleic acids comprising:
a) "Zm": a nucleic acid derived from and specific for *Zea mays* taxon, preferably *Zea mays* ssp. *mays,*
b) "Bn": a nucleic acid derived from and specific for *Brassica napus* taxon,
c) "Gm": a nucleic acid derived from and specific for *Glycine max* taxon,
d) "p35S": a nucleic acid derived from the 35S promoter of Cauliflower Mosaic Virus,
e) "tNOS": a nucleic acid derived from the 3' terminator of the *Agrobacterium tumefaciens* nopaline synthetase gene,
f) "Cry1Ab": a nucleic acid derived from the crystal protein gene *Cry1Ab* of *Bacillus thuringiensis,*
g) "PAT/bar": a nucleic acid derived from the phosphinothricin acetyltransferase (PAT) gene *bar* of *Streptomyces hygroscopicus,*
h) "PAT/pat": a nucleic acid derived from the phosphinothricin acetyltransferase (PAT) gene pat of *Streptomyces viridochromogenes,* and
i) "CP4-EPSPS": a nucleic acid derived from the 5-Enol-pyruvylshikimate-3-phosphate synthase *EPSPS* gene from *Agrobacterium* sp. CP4; and
(2) concluding the presence or absence in the sample of material derived from one or more transgenic plant events chosen from the group comprising: events Bt176, Bt11, Bt10, MON810, MON863, TC1507, NK603, T25, GA21, DAS-59122, crosses thereof, and related events thereof; events Topas 19/2, MS1, RF1, RF2, RF3, MS8, GT73, T45, Liberator pHoe6/Ac, GS40/90pHoe6/Ac, crosses thereof including MS1/RF1, MS1/RF2, MS8/RF3, and related events thereof; events MON 40-3-2, MON89788, A2704-12, A5547-127, crosses thereof, and related events thereof.

2. The method according to claim 1,
- wherein step (1) further comprises detecting the presence or absence in the sample of nucleic acid j) "mCry3A": a nucleic acid derived from the modified crystal protein gene *Cry*3*A* of Bacillus thuringiensis, and the group of events as defined in step (2) further comprises maize event MIR604, crosses thereof with other maize events, and events related to MIR604; and/or
- wherein step (1) further comprises detecting the presence or absence in the sample of one or both nucleic acids k) "cordapA": a nucleic acid derived from the lysine-insensitive dihydrodipicolinate synthase (cDHDPS) gene *cordapA* of *Corynebacterium glutamicum* and I) "GIb1": a nucleic acid derived from the GIb1 promoter of maize, and the group of events as defined in step (2) further comprises maize event LY038, crosses thereof with other maize events, and events related to LY038; and/or
- wherein step (1) further comprises detecting the presence or absence in the sample of nucleic acid m) "Cry3Bb1": a nucleic acid derived from the crystal protein gene *Cry3Bb1* of Bacillus thuringiensis, and the group of events as defined in step (2) further comprises maize event MON88017, crosses thereof with other maize events, and events related to MON88017; and/or
- wherein step (1) further comprises detecting the presence or absence in the sample of nucleic acid n) "Bxn": a nucleic acid derived from the nitrilase gene *Bxn* of *Klebsiella pneumoniae* ssp. *ozaenae*, and the group of events as defined in step (2) further comprises oilseed rape event OXY235, crosses thereof with other oilseed rape events, and events related to OXY235; and/or
- wherein step (1) further comprises detecting the presence or absence in the sample of nucleic acid o) "Or": a nucleic acid derived from and specific for *Oryza sativa* taxon, and the group of events as defined in step (2) further comprises one, more than one, or all of the rice events LL62, LL06 and LL601, crosses thereof, and events related thereto; and/or
- wherein step (1) further comprises detecting the presence or absence in the sample of nucleic acid p) "Bv": a nucleic acid derived from and specific for *Beta vulgaris* taxon, and the group of events as defined in step (2) further comprises one, more than one, or all of the sugar beet events T120-7, H7-1 and A5-15, crosses thereof, and events related thereto; and/or
- wherein step (1) further comprises detecting the presence or absence in the sample of nucleic acid q) "Gs": a nucleic acid derived from and specific for *Gossypium* taxon; and the group of events as defined in step (2) further comprises one, more than one, or all of the cotton events LL cotton 25, MON 1445, crosses thereof, and events related thereto; and optionally, wherein step (1) further comprises detecting the presence or absence in the sample of one or both of nucleic acids r) "Cry1Ac": a nucleic acid derived from the crystal protein gene *Cry1Ac* of *Bacillus thuringiensis* and s) "Cry2Ab2": a nucleic acid derived from the crystal protein gene *Cry2Ab2* of *Bacillus thuringiensis,* and the group of events as defined in step (2) further comprises one or both of cotton events MON 531, MON15985, crosses thereof with other cotton events, and events related thereto; and/or
- wherein step (1) further comprises detecting the presence or absence in the sample of nucleic acid t) "St": a nucleic acid derived from and specific for *Solanum tuberosum* taxon, and the group of events as defined in step (2) further comprises the potato event EH92-527-1 and events related thereto; and optionally wherein step (1) further comprises detecting the presence or absence in the sample of nucleic acid u) "GBSS": a nucleic acid derived from the granule bound starch synthase gene *Gbss* of *Solanum tuberosum.*

3. The method according to any of claims 1 or 2, wherein the nucleic acid a) "Zm" derived from and specific for *Zea mays* taxon is derived from the endogenous alcohol dehydrogenase I gene (*adh-1*) of *Zea mays.*

4. The method according to any of claims 1 to 3, wherein the nucleic acid b) "Bn" derived from and specific for *Brassica napus* taxon is derived from the endogenous acetyl-CoA carboxylase gene (*acc*) or the endogenous cruciferin gene of *Brassica napus.*

5. The method according to any of claims 1 to 4, wherein the nucleic acid c) "Gm" derived from and specific for *Glycine max* taxon is derived from the endogenous lectin gene (*lec*) of *Glycine max.*

6. The method according to any of claims 2 to 5, wherein the nucleic acid o) "Or" derived from and specific for *Oryza sativa* taxon is derived from the endogenous phospholipase D gene (*lec*) of *Oryza sativa.*

7. The method according to any of claims 1 to 6, wherein step (1) further comprises detecting the presence or absence in the sample of a generic plant-derived nucleic acid, preferably derived from the chloroplastic small subunit of Rubisco gene or from the CHL-tRNA synthetase gene.

8. The method according to any of claims 1 to 7, wherein the presence or absence of the said nucleic acids in the sample is detected using amplification of amplicons comprised in the respective nucleic acids, preferably using PCR, more preferably using real-time PCR.

9. The method according to claim 8, wherein the said amplicons are less than 300 bp, preferably less than 200 bp, more preferably less than 150 bp, even more preferably less than 100 bp, and yet more preferably less than 90 bp.

10. The method according to claim 9, wherein the said amplicons are between about 50 bp and about 90 bp, preferably between about 60 bp and about 80 bp, and more preferably between about 65 bp and about 75 bp.

11. The method according to any of claims 8 to 10, wherein the amplification products are detected using a detection method that is substantially sequence non-specific, preferably using a DNA-binding fluorescent dye, more preferably using SYBR Green or PicoGreen.

12. The method according to any of claims 8 to 11, wherein the specificity of the amplification products is further verified, preferably using melting curve analysis (Tm) and/or size determination by gel elecrophoresis.

13. The method according to any of claims 8 to 12, wherein the amplification of amplicons within the said nucleic acids uses primer pairs listed in the following table:
| **Nucleic acid** | Primers |
|---|---|
| "Zm" | Fwd: 5' CgTCgTTTCCCATCTCTTCCTCC 3' (SEQ ID NO: 1) |
| (adh-1) | Rev: 5' CCACTCCgAgACCCTCAgTC 3' (SEQ ID NO: 2) |
| "Bn" | Fwd: 5' GGCGCAGCATCGGCT 3' (SEQ ID NO: 3) |
| (ACC) | Rev: 5' GAGAATGAGGAGGACCAAGCTC 3' (SEQ ID NO: 4) |
| "Gm" | Fwd: 5' CATTACCTATgATgCCTCCACC 3' (SEQ ID NO: 5) |
| (lectin) | Rev: 5' AAgCACgTCATgCgATTC 3' (SEQ ID NO: 6) |
| | Rev': 5' AAgCACgTCATgCgATTCC 3' (SEQ ID NO: 49) |
| "p35S" | Fwd: 5'-GACAGTGGTCCCAAAGATGG-3' (SEQ ID NO: 7) |
| | Rev: 5'-GTCTTGCGAAGGATAGTGGG-3' (SEQ ID NO: 8) |
| "tNOS" | Fwd: 5'-GATTAGAGTCCCGCAATTATACATTTAA-3' (SEQ ID NO: 9) |
| ("TNOS-D") | Rev: 5'-TTATCCTAGKTTGCGCGCTATATTT-3' (SEQ ID NO: 10) |
| "Cry1 Ab" | Fwd: 5'-ACCGGTTACACTCCCATCGA-3' (SEQ ID NO: 11) |
| | Rev: 5'-CAGCACCTGGCACGAACTC-3' (SEQ ID NO: 12) |
| "PAT/bar" | Fwd: 5'-CGTCAACCACTACATCGAGACAA-3' (SEQ ID NO: 13) |
| | Rev: 5'-GTCCACTCCTGCGGTTCCT-3' (SEQ ID NO: 14) |
| "PAT/pat" | Fwd: 5'-CCGCGGTTTGTGATATCGTT-3' (SEQ ID NO: 15) |
| | Rev: 5'-TCTTGCAACCTCTCTAGATCATCAA-3' (SEQ ID NO: 16) |
| "CP4-EPSPS" | Fwd1: 5'-GGCTCTGAGCTTCGTCCTCCTAAGG-3' (SEQ ID NO: 17) |
| | Fwd1': 5'-GGCTCTGAGCTTCGTCCTCTTAAGG-3' (SEQ ID NO: 50) |
| | Fwd2: 5'-ATCAGTGGCTACAGCCTGCAT-3' (SEQ ID NO: 18) |
| | Rev: 5'-GAATGCGGACGGTTCCGGAAAG-3' (SEQ ID NO: 19) |
| "Or" | Fwd: 5' GCTTAGGGAACAGGGAAGTAAAGTT 3' (SEQ ID NO: 20) |
| | Fwd': 5' GCTTAGGGAACAGGGAAGTAAAGT 3' (SEQ ID NO: 51) |
| | Rev: 5' CTTAGCATAGTCTGTGCCATCCA 3' (SEQ ID NO: 21) |
| "CP4-EPSPS" | Fwd: 5' GCATGCTTCACGGTGCAA 3' (SEQ ID NO: 22) |
| | Rev: 5' GGACCTGTGGGAGATAGACTTGTC 3' (SEQ ID NO: 23) |
| "Bn" | Fwd: 5' CAGCTCAACAGTTTCCAAACGA 3' (SEQ ID NO: 24) |
| (cruciferin) | Rev: 5' CGACCAGCCTCAGCCTTAAG 3' (SEQ ID NO: 25) |
| generic plant | Fwd: 5' AGGTCTAADGGRTAAGCTAC 3' (SEQ ID NO: 26) |
| (Rbcl) | Rev: 5' AGYCTTGATCGTTACAAAGG 3' (SEQ ID NO: 27) |
| "tNOS" | Fwd: 5' CGTTCAAACATTTGGCAATAAAG 3' (SEQ ID NO: 28) |
| (TNOS-L) | Rev: 5' AAATGTATAATTGCGGGACTCTAATC 3' (SEQ ID NO: 29) |
| "Cry1Ab" | Fwd: 5' GACATCATCTGGGGYATCTT 3' (SEQ ID NO: 30) |
| | Rev: 5' GCGCTGTTCATGTCGTTGAA 3' (SEQ ID NO: 31) |
| "Cry1Ab" | Fwd: 5' ACGCCTTCCTGGTGCAAA 3' (SEQ ID NO: 47) |
| | Rev: 5' CCTGGTTCCTGGCGAACTC 3' (SEQ ID NO: 48) |

14. The method according to any of claims 1 to 13, wherein the sample comprises plants or parts thereof, including flowers, tepals, petals, sepals, anthers, pollen, seeds, fruits, pericarp, pods, leaves, petioles, stems, roots, rhizomes, stolons, tubers or shoots, or portions thereof, plant cells, plant protoplasts and/or plant tissues, and/or plant-derived material, preferably food or feed material, including processed food or feed material.

15. The method according to any of the preceding claims, wherein the step (2) comprises the steps of:
(i) defining a set *"G_{SAM}"* consisting of nucleic acids detected in step (1) as being present in the sample;
(ii) defining one or more set of interest ("Gₓ") chosen from the group of sets comprising or consisting of sets *"G_{Bt176}" "G_{Bt11}", "G_{Bt10}", "G_{MON810}", "G_{MON863}", "G*_{*TC150*/}*, "*G*_{NK603}", "*G*_{T25}", "*G*_{GA21}",* "G*_{DAS-59122}",* "G*_{MIR604}", "*G*_{LY038}", "*G*_{MON88017}"_{,}"*G*_{Topas} 19*/*2", "*G*_{MS1}", "*G*_{RF1}",* "G*_{RF2}*", *"*G_{*MS1*/}*_{RF1}", "*G_{*MS1*/}*_{RF2}",* "G*_{MS8}*", "G*_{RF3}*", "G_{*MS8*/*RF3*}", "G*_{GT73}*", "G*_{T45}*", *"*G_{*Liberator pHoe6*/}*_{Ac}" "*G_{*GS40*/*90pHoe6*/}*_{AC}", "*G*_{OXY235}"; "*G*_{MON40-3-2}", "*G*_{MON89788}", "*G*_{A2704-12}", "*G*_{A5547-127}"*, "G*_{LL62}*", *"*G*_{LL06}", "*G*_{LL601}",* "G*_{T120-7}*", "G*_{H7-1}*"_{,} *"*G*_{A5-15}", "*G*_{LL cotton 25}*", *"*G*_{MON1445}"_{,}* "G_{*MOM53*1}", *"*G*_{MON15985}"* and "G_{*EH92-527*-*1*}"; corresponding to the respective one or more transgenic plant events of interest ("X") chosen from the group comprising or consisting of events Bt176, Bt11, Bt10, MON810, MON863, TC1507, NK603, T25, A21, DAS-59122, MIR604, LY038, MON88017,Topas 19/2, MS1, RF1, RF2, MS1/RF1, MS1/RF2, MS8, RF3, MS8/RF3, GT73, T45, Liberator pHoe6/Ac, GS40/90pHoe6/Ac, OXY235; MON40-3-2, MON89788, A2704-12, A5547-127, LL62, LL06, LL601, T120-7, H7-1, A5-15, LL cotton 25, MON1445, MON531, MON15985 and EH92-527-1, wherein:
- G*_{Bt176}* ∈ {Zm; p35S; Cry1Ab; PAT/bar},
- G*_{Bt11}* ∈ {Zm; p35S; tNOS; Cry1Ab; PAT/pat},
- G*_{Bt10}* ∈ {Zm; p35S; tNOS; Cry1Ab; PAT/pat},
- G*_{MON810}* ∈ {Zm; p35S; tNOS; CrylAb},
- G_{MON863} ∈ {Zm; p35S; tNOS},
- G*_{TC1507}* ∈ {Zm; p35S; PAT/pat},
- G*_{NK603}* ∈ {Zm; p35S; tNOS; CP4-EPSPS},
- G_{T25} ∈ {Zm; p35S; PAT/pat},
- G*_{GA21}* ∈ {Zm; tNOS},
- G_{DAS-59122} ∈ {Zm; p35S; PAT/bar},
- G*_{MIR604}* ∈{Zm; tNOS; mCry3A},
- G*_{LY038}* ∈ {Zm; p35S; tNOS; cordapA; Glb1},
- G*_{MON88017}* ∈ {Zm; p35S; tNOS; CP4-EPSPS; Cry3Bb1},
- G_{*Topas 19*/*12*} ∈ {Bn; p35S; PAT/pat},
- G*_{MS1}* ∈ {Bn; tNOS; PAT/bar},
- G*_{RF1}* ∈ {Bn; tNOS; PAT/bar},
- G*_{RF2}* ∈ {Bn; tNOS; PAT/bar},
- G_{*MS1*/*RF1*} ∈ {Bn; tNOS; PAT/bar},
- G_{*MS1*/*RF2*} ∈ {Bn; tNOS; PAT/bar},
- G*_{MS8}* ∈ {Bn; tNOS; PAT/bar},
- G*_{RF3}* ∈ {Bn; tNOS; PAT/bar},
- G_{*MS8*/*RF3*} ∈ {Bn; tNOS; PAT/bar},
- G*_{GT73}* ∈{Bn; CP4-EPSPS},
- G*_{T45}* ∈ {Bn; p35S; PAT/pat},
- G_{*Liberator pHoe6*/*Ac*} ∈ {Bn; p35S; PAT/pat},
- G_{*GS40*/*90pHoe6*/*Ac*} ∈ {Bn; p35S; PAT/pat},
- G_{O*XY235*} ∈ {Bn; p35S; Bxn};
- G*_{MON40-3-2}* ∈ {Gm; p35S; tNOS; CP4-EPSPS},
- G*_{MON89788}* ∈ {Gm; CP4-EPSPS}
- G*_{A2704-12}* ∈ {Gm; p35S; PAT/pat},
- G*_{A5547-127}* ∈ {Gm; p35S; PAT/pat},
- G*_{LL62}* ∈ {Or; p35S; PAT/bar},
- G*_{LL06}* ∈ {Or; p35S; PAT/bar}
- G*_{LL601}* ∈ {Or; p35S; tNOS; PAT/bar},
- G*_{T120-7}* ∈ {Bv; p35S; PAT/pat},
- G*_{H7-1}* ∈ {Bv; p35S; CP4-EPSPS},
- G*_{A5-15}* ∈ {Bv; p35S; tNOS; CP4-EPSPS},
- G*_{LL cotton 25}* ∈ {Gs; p35S; tNOS; PAT/bar},
- G*_{MON1445}* ∈ {Gs; p35S; tNOS; CP4-EPSPS},
- G*_{MON531}* ∈ {Gs; p35S; tNOS; cry1Ac}
- G*_{MON15985}* ∈ {Gs; p35S; tNOS; cry1Ac; cry2Ab2}, and
- G*_{EH92-527-1}* ∈ {St; tNOS; Gbss};
(iii) performing for each set of interest G*ₓ* logical operations:
- if Gx equals G*_{SAM}* (G*ₓ* = G*_{SAM}*)*,* then material derived from the transgenic plant event *X* or from a cross thereof, or from an event related thereto, is potentially present in the sample;
- if Gₓ is a proper subset of G*_{SAM}* (G*ₓ* ⊂ G*_{SAM}*), then material derived from transgenic plant event *X* or from a cross thereof, or from an event related thereto, is potentially present in the sample;
- if G*ₓ* does not equal G*_{SAM}* and G*ₓ* is not a proper subset of G*_{SAM},* (G*ₓ* ≠ G*_{SAM}* and G*ₓ* ⊄ G*_{SAM}),* then material derived from transgenic plant event *X* or from a cross thereof, or from an event related thereto, is absent from the sample.

16. The method according to claim 15, wherein if Gₓ equals G_{SAM}, and if no one set or the sum of two or more sets chosen from the group comprising or consisting of G*_{Bt176},* G*_{Bt11}.* G*_{Bt10},* G*_{MON810},* G_{MON863}, G*_{TC1507},* G*_{NK6033}* G*_{T253}* G*_{GA213}* G_{DAS-59122}, G*_{MIR604},* G*_{LY038},* G_{MON88017},G_{Topas 19/2}, G_{MS1}, G*_{RF1},* G_{RF2}, G_{*MS1*/}*_{RF1},* G_{*MS1*/}*_{RF2},* G_{MS8}, G*_{RF3},* G_{MS8/RF3}, G*_{GT73},* G*_{T45},* G_{*Liberator pHoe6*/}*_{Ac},* G_{*GS40l90pHoe6*/}*_{Ac},* G*_{OXY235},* G*_{MON40-3-2},* G_{MON89788}, G*_{A2704-12},* G*_{A5547-127,}* G_{LL62,} G*_{LL06,}* G*_{LL601},* G*_{T120-7}*, G_{H7-1}, G*_{A5-15},* G_{*LL cotton* 25}, G*_{MON1445},* G*_{MON531},* G*_{MON15985}* other than Gₓ equals Gₓ, then material derived from transgenic plant event *X* or from a cross thereof, or from an event related thereto, is present in the sample.

17. The method according to claim 15, wherein nucleic acids chosen from the group comprising or consisting of "Zm", "Bn", "Gm", "p35S", "tNOS", "Cry1Ab", "PAT/bar", "PAT/pat", "CP4-EPSPS", "mCry3A", "cordap A", "Glb1", "Cry3Bb1", "Bxn", "Or", "Bv", "Gs", "Cry1Ac", "Cry2ab2", "St" and "Gbss" are assigned respective unique values "V*_{zm}*", *"V_{Bn}",* "V*_{Gm}*", *"*V*_{p35S}", "*V*_{tNOS}", "*V*_{Cry1Ab}", "*V_{*PAT*/}*_{bar}", "*V_{*PAT*/}*ₚₐₜ", "*V*_{CP4-EPSPS}", "*V*_{mCry3A}", "*V*_{cordap A}", "*V*_{Glb1}"*, *"*V*_{Cry3Bb1}", "*V*_{Bxn}",* "V*_{Or}", "*V*_{Bv}",* "V*_{Gs}*", "V*_{Cry1Ac}*", *"*V*_{Cry2ab2}", "*V*_{St}"* and *"*V*_{Gbss}"*; wherein each set of interest G*ₓ* is assigned a value "VG*ₓ*" chosen from the group comprising consisting of values *"*VG*_{Bt176}", "*VG*_{Bt11}",* "VG*_{Bt10}",* "VG*_{MON810}",* "VG*_{MON863}*", "VG*_{TC1507}*", "VG*_{NK603}*", "V"G*_{T25}*", "VG*_{GA21}*", *"*VG*_{DAS-59122}", "*VG*_{MIR604}*"*,* "VG*_{LY038}*", *"*VG*_{MON88017}", "*VG_{*Topas 19*/}*₂",* "VG*_{MS1}*", "VG*_{RF1}*", "VG*_{RF2}*", *"*VG_{*MS1*/}*_{RF1}", "*VG_{*MS1*/}*_{RF2}",* "VG*_{MS8}*", "VG*_{RF3}*", *"*VG_{*MS8*/}*_{RF3}",* "VG*_{GT73}*", "VG*_{T45}*", *"*VG_{*Liberator pHoe6*/}*_{Ac}"*, "VG_{*GS40*/*90pHoe6*/*Ac*}", *"*VG*_{OXY235}",* "VG*_{MOM40-3-2}*", *"*VG*_{MON89788}", "*VG*_{A2704-12}",* "VG*_{A5547-127}*", "VG*_{LL62}"*, *"*VG*_{LL06}",* "VG_{*LL601"*,} "VG*_{T120-7}*", "VG*_{H7-1}*", "VG*_{A5-15}*", *"*VG*_{LL} cotton 25*", *"*VG*_{MON1445}", "*VG*_{MON531}",* "VG*_{MON15985}*" and "VG_{EH92}-₅₂₇₋₁", wherein:
- VG*_{Bt176}* = V*_{Zm}* × V*_{p35S}* × V*_{Cry1Ab}* × V_{*PAT*/}*_{bar},*
- VG*_{Bt11}* = V*_{Zm}* × V*_{p35S}* × V*_{tNOS}* × V*_{Cry1Ab}* × V_{*PAT*/*pat*},
- VG*_{Bt10}* = V*_{Zm}* × V*_{p35S}* × V*_{tNOS}* × V*_{Cry1Ab}* × V_{*PAT*/*pat*},
- VG*_{MON810} =* V*_{Zm}* × V*_{p35S}* × V*_{tNOS}* × V*_{Cry1Ab}*,
- VG*_{MOM863}* = V*_{Zm}* × V*_{p35S}* × V*_{tNOS}*,
- VG*_{TC1507}* = V*_{Zm}* × V*_{p35S}* × V_{*PAT*/}*ₚₐₜ,*
- VG*_{MK603}* = V*_{Zm}* × *V_{p35S}* × V*_{tNOS}* × V*_{CP4-EPSPS},*
- VG*_{T25}* = V*_{Zm}* × V*_{p35S}* × V_{*PAT*/*pat*},
- VG*_{GA21}* = V*_{Zm}* × V*_{tNOS}*,
- VG*_{DAS-59122}* = V*_{Zm}* × V*_{p35S}* × V_{*PAT*/*bar*},
- VG*_{MIR604}* = V*_{Zm}* × V*_{tNOS}* × V*_{mCry3A} ,*
- VG*_{Ly038}* = V*_{Zm}* × V*_{p35S}* × V*_{tNOS}* × V*_{cordapA}* × V*_{Glb1}*,
- VG*_{MON88017}* = V*_{Zm}* × V*_{p35S}* × V*_{tNOS}* × V*_{CP4-EPSP}* × *V_{Cry3Bb1},*
- VG_{*Topas 19*/*2*} = V*_{Bn}* × V*_{p35S}* × V_{*PAT*/}*ₚₐₜ,*
- VG*_{MS1}* = V*_{Bn}* × V*_{tNOS}* × V_{*PAT*/}*_{bar},*
- VG*_{RF1}* = V*_{Bn}* × V*_{tNOS}* × V_{*PAT*/*bar*},
- VG*_{RF2}* = V*_{Bn}* × V*_{tNOS}* × V_{*PAT*/*bar*},
- VG*_{MS8}* = V*_{Bn}* × V*_{tNOS}* × V_{*PAT*/*bar*},
- VG*_{RF3}* = V*_{Bn}* × V*_{tNOS}* × V_{*PAT*/}*_{bar},*
- VG_{*MS1*/*RF1*} = V*_{Bn}* × V*_{tNOS}* × V_{*PAT*/*bar*},
- VG_{*MS1*/*RF2*} = V*_{Bn}* × V*_{tNOS}* × V_{*PAT*/*bar*},
- VG_{*MS8*/*RF3*} = V*_{Bn}* × V*_{tNOS}* × V_{*PAT*/*bar*},
- VG*_{GT73}* = V*_{Bn}* × V*_{CP4-EPSPS,}*
- VG*_{T45} =* V*_{Bn}* × V*_{p35S}* × V_{*PAT*/}*ₚₐₜ,*
- VG_{*Liberator pHoe6*/}*_{Ac} =* V*_{Bn}* × V*_{p35S}* × V_{*PAT*/*pat*},
- VG_{*GS40*/*90PHoe6*/*Ac*} = V*_{Bn}* × V*_{p35S}* × V_{*PAT*/}*ₚₐₜ,*
- VG*_{OXY235} =* V*_{Bn}* × V*_{p35S}* × V*_{Bxn},*
- VG*_{MON40-3-2}* = V*_{Gm}* × V*_{p35S}* × V*_{tNOS}* × V*_{CP4-EPSPS},*
- VG*_{MON89788}* = V*_{Gm}* × V*_{CP4-EPSPS3}*,
- VG*_{A2704-12} =* V*_{Gm}* × V*_{p35S}* × V_{*PAT*/*pat,*}
- VG*_{A5547-127} =* V*_{Gm}* × V*_{p35S}* × V_{*PAT*/*pat*},
- VG*_{LL62} =* V*_{Or}* × V*_{p35S}* × V_{*PAT*/}*_{bar},*
- VG*_{LL06}* = *V_{Or}* × V*_{p35S}* × V_{*pAT*/}*_{bar},*
- VG*_{LL601}* = V*_{Or}* × V*_{p35S}* × Vt*_{NOS}* × V_{*PAT*/}*_{bar},*
- VG*_{T120-7}* = V*_{Bv}* × V*_{p35S}* × V_{*PAT*/}*ₚₐₜ,*
- VG*_{H7-1}* V*_{Bv}* × V*_{p35S}* × V*_{CP4-EPSPS}*,
- VG*_{A5-15}* = V*_{Bv}* × V*_{p35S}* × V*_{tNOS}* × V*_{CP4-EPSPS},*
- VG_{L*L cotton* 25} = V_{Gs} × V*_{p35S}* × V_{tNOS} × V_{*PAT*/}*_{bar},*
- VG*_{MON1445}* = V_{Gs} × V_{p35S} × V_{tNOS} × V*_{CP4-EPSPS},*
- VG_{MON531} *=* V_{Gs} × V_{p35S} × V_{tNOS} × V*_{Cry1Ac},*
- VG*_{MON15985}* = V*_{Gs}* × V*_{p35S}* × V*_{tNOS}* × V*_{Cry1Ac}* × V*_{Cry1Ac}*,
- Or VG*_{EH92-527-1}* = V *_{St}* × V*_{tNOS}* × V*_{Gbss},*
and wherein the set G*_{SAM}* is assigned a value "VG*_{SAM}*" which is a multiple of the unique values assigned to the nucleic acids detected in step (1) as being present in the sample; and wherein step (iii) comprises performing for each set of interest G*ₓ* logical operations:
- if VG*_{SAM}* / VG*ₓ* equals 1, then material derived from transgenic plant event *X* or from a cross thereof, or from an event related thereto, is potentially present in the sample;
- if VG*_{SAM}* / VG*ₓ* equals one or a multiple of two or more values chosen from a group comprising or consisting of values V*_{zm},* V*_{Bn},* V*_{Gm},* V*_{p35S},* V*_{tNOS},* V*_{cry1Ab},* V_{*PAT*/}*_{bar},* V_{*PAT*/}*ₚₐₜ,* V*_{CP4-EPSPS},* V*_{mCry3A} ,* V*_{cordap A},* V*_{Glb1},* V*_{Cry3Bb1},* V*_{Bxn},* V*_{Or},* V*_{Bv},* V*_{Gs}*, V*_{Cry1Ac},* V *_{Cry2ab2}*, V*_{St}* and V*_{Gbss}*, then material derived from transgenic plant event *X* or from a cross thereof, or from an event related thereto, is potentially present in the sample;
- if VG*_{SAM}* / VG*ₓ* does not equal 1 and does not equal one value or a multiple of two or more values chosen from a group comprising or consisting of values V*_{Zm},* V*_{Bn},* V*_{Gm}*, V*_{p35S3}*, V*_{tNOS},* V*_{Cry1Ab}*, V_{*PAT*/}*_{bar},* V*_{PATlpat},* V*_{CP4-EPSPS},* V*_{mCry3A},* V*_{cordap A},* V*_{Glb1},* V*_{Cry3Bb1}*, V*_{Bxn},* V*_{Or},* V*_{Bv},* V*_{Gs}*, V*_{Cry1Ac},* V*_{Cry2ab2},* V*_{St}* and V*_{Gbss},* then material derived from transgenic plant event *X* or from a cross thereof, or from an event related thereto, is absent from the sample.

18. The method according to claim 17, wherein if VG_{SAM} / VGₓ equals 1, and if no one value or the multiple of two or more values of sets chosen from a group comprising or consisting of: VG_{Bt176}, VG*_{Bt11},* VG*_{Bt10},* VG_{MON810}, VG_{MON8633} VG_{TC1507}, VG_{NK6033} VG_{T25}, VG*_{GA21},* VG_{DAS-59122}, VG*_{MIR604},* VG*_{LY038},* VG*_{MON88017},* VG*_{Topas 1912},* VG*_{MS1},* VG*_{RF1},* VG*_{RF2},* VG_{*MS1*/}*_{RF1},* VG_{*MS1*/}*_{RF2},* VG_{MS8}, VG*_{RF3},* VG_{*MS8*/}*_{RF3},* VG*_{GT73},* VG_{T45}, VG_{*Liberator pHoe6*/}*_{Ac},* VG_{*GS40*/*90pHoe6*/*Ac,*} VG*_{OXY235},* VG*_{MON40-3-2},* VG*_{MON89788},* VG*_{A2704-12},* VG*_{A5547-127},* VG*_{LL62},* VG*_{LL06},* VG*_{LL601},* VG_{T120-7}, VG*_{H7-1},* VG_{A5-15}, VG*_{LL cotton 25},* VG*_{MON1445},* VG*_{MON5313}* VG*_{MON15985}* and VG*_{EH92-527-1}* Other than VGₓ equals VGₓ, then material derived from transgenic plant event *X* or from a cross thereof, or from an event related thereto, is present in the sample.

19. The method according to any of claims 17 or 18, wherein values V*_{zm},* V*_{Bn},* V*_{Gm},* V*_{p35S}*, V*_{tNOS},* V*_{Cty1Ab},* V_{*PAT*/}*_{bar},* V_{*PAT*/}*ₚₐₜ,* V*_{CP4-EPSPS},* V*_{mCry3A},* V*_{cordap A},* V*_{Glb1},* V*_{Cry38b1},* V*_{Bxn},* V*_{Or},* V *_{Bv},* V_{Gs}, V*_{Cry1Ac},* V*_{Cry2ab2},* V*ₛₜ* and V*_{Gbss}* are unique prime numbers, and wherein step (iii) comprises performing for each set of interest Gₓ logical operations,
- if VG_{SAM} / VGₓ equals 1, then material derived from transgenic plant event *X* or from a cross thereof, or from an event related thereto, is potentially present in the sample;
- if VG_{SAM} / VGₓ is an integer greater than 1, then material derived from transgenic plant event *X* or from a cross thereof, or from an event related thereto, is potentially present in the sample;
- if VG*_{SAM}* / VG*ₓ* is not an integer, then material derived from transgenic plant event *X* or from a cross thereof, or from an event related thereto, is absent from the sample.

20. The method according to any of claims 1 to 19, wherein step (2) is carried out by a computing device.

21. Primers and primer pairs as defined in claim 13.

22. Amplification products obtainable by using primer pairs as defined in claim 13; recombinant vectors and plasmids comprising the said amplification products; recombinant microorganisms transformed with the said vectors and/or plasmids.

23. Recombinant *E*. *coli* as deposited under the Budapest Treaty with the Belgian Coordinated Collections of Microorganisms (BCCM) on January 10, 2007 under LMBP accession numbers LMBP 5452, LMBP 5453, LMBP 5454, LMBP 5455, LMBP 5456, LMBP 5457, LMBP 5458, LMBP 5459 and LMBP 5460, on March 6, 2007 under LMBP accession number LMBP 5451, and on April 19, 2007 under LMBP accession numbers LMBP 5587, LMBP 5588, LMBP 5589 and LMBP 5590.

24. Isolated recombinant plasmids obtainable from any of recombinant E. *coli* bacteria according to claim 23, or isolated inserts of the said plasmids, preferably isolated EcoRI-EcoRI inserts thereof.

25. A recombinant microorganism transformed with a plasmid according to claim 24.

26. A kit for examining a sample for the potential presence or absence of material derived from one or more transgenic plant events, the kit comprising primers, and preferably primer pairs, suitable for amplification of one or more, preferably all, nucleic acids as defined in any of claims 1 to7.

27. The kit according to claim 26, comprising one or more primers, and preferably primer pairs, preferably all primers or primer pairs, as defined in claim 13.

28. The kit according to any of claims 26 or 27, further comprising one or more nucleic acids suitable as a positive control for the amplification of nucleic acids according to any of claims 1 to 7.

29. The kit of any of claims 26 to 28, comprising one or more recombinant E. coli bacteria as defined in claim 23, recombinant microorganisms as defined in claim 25, isolated recombinant plasmids or inserts according to claim 24.

30. The kit of any of claims 26 to 29 further comprising a data carrier comprising instructions for a programmable computing device to carry out step (2) of the method according to any of claims 1 to 20.
